(19) Europäisches Patentamt / European Patent Office / Office européen des brevets

(11) **EP 2 478 371 B1**

(12) **EUROPEAN PATENT SPECIFICATION**

(45) Date of publication and mention
of the grant of the patent:
**04.05.2016 Bulletin 2016/18**

(51) Int Cl.:
***G01N 33/68*** (2006.01)

(21) Application number: **10752842.4**

(22) Date of filing: **16.09.2010**

(86) International application number:
**PCT/EP2010/063637**

(87) International publication number:
**WO 2011/033034 (24.03.2011 Gazette 2011/12)**

(54) **Multimarker panel for left ventricular hypertrophy**

Multimarker-Panel für Linksherzhypertrophie

Panneau à plusieurs marqueurs pour l'hypertrophie ventriculaire

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **17.09.2009 EP 09011888**

(43) Date of publication of application:
**25.07.2012 Bulletin 2012/30**

(73) Proprietors:
• **Roche Diagnostics GmbH**
**68305 Mannheim (DE)**
Designated Contracting States:
**DE**
• **F.Hoffmann-La Roche AG**
**4070 Basel (CH)**
Designated Contracting States:
**AL AT BE BG CH CY CZ DK EE ES FI FR GB GR HR HU IE IS IT LI LT LU LV MC MK MT NL NO PL PT RO SE SI SK SM TR**

(72) Inventors:
• **HESS, Georg**
**55130 Mainz (DE)**
• **HORSCH, Andrea**
**68259 Mannheim (DE)**
• **ZDUNEK, Dietmar**
**82327 Tutzing (DE)**

(74) Representative: **Herzog, Fiesser & Partner**
**Patentanwälte PartG mbB**
**Patentanwälte**
**Dudenstrasse 46**
**68167 Mannheim (DE)**

(56) References cited:
**EP-A1- 2 037 275      EP-A1- 2 090 891**
**WO-A2-2006/034356**

• **ARTEAGA ET AL: "Plasma amino-terminal pro-B-type natriuretic peptide quantification in hypertrophic cardiomyopathy" AMERICAN HEART JOURNAL, MOSBY- YEAR BOOK INC, US, vol. 150, no. 6, 1 December 2005 (2005-12-01), pages 1228-1232, XP005199296 ISSN: 0002-8703**
• **LOWBEER ET AL: "Serum cardiac troponin T, troponin I, plasma BNP and left ventricular mass index in professional football players" JOURNAL OF SCIENCE AND MEDICINE IN SPORT, SPORTS MEDICINE AUSTRALIA, BELCONNEN, AU, vol. 10, no. 5, 24 August 2007 (2007-08-24), pages 291-296, XP022211854 ISSN: 1440-2440**
• **XU JIAN ET AL: "GDF15/MIC-1 functions as a protective and antihypertrophic factor released from the myocardium in association with SMAD protein activation." CIRCULATION RESEARCH 17 FEB 2006, vol. 98, no. 3, 17 February 2006 (2006-02-17), pages 342-350, XP002565988 ISSN: 1524-4571**

**Description**

An aim of modern medicine is to provide personalized or individualized treatment regimens. Those are treatment regimens which take into account a patient's individual needs or risks. Personalized or individual treatment regimens shall be even taken into account for measures where it is required to decide on potential treatment regimens.

Left ventricular hypertrophy (LVH) is a condition in which the walls of the ventricle thicken. This phenomenon can result from various reasons. For example, it is found in athlete's hearts (athletic heart syndrome) as adaptation to enhanced needs for blood supply and does not require any treatment.

Left ventricular hypertrophy is also found in patients suffering from arterial hypertension, i.e. high blood pressure being a disease requiring treatment. This form is generally referred to as "hypertensive left ventricular hypertrophy". Furthermore, left ventricular hypertrophy can be caused by aortic stenosis, which in the present application will generally be referred to as "hypertrophy associated with aortic stenosis". Both hypertensive left ventricular hypertrophy and hypertrophy associated with aortic stenosis are comprised within the term "pressure overload hypertrophy" as used in the present application.

Arterial hypertension places increased tension on the left ventricular myocardium that is manifested as stiffness and hypertrophy. Independently thereof, atherosclerosis develops within the coronary vessels as a consequence of hypertension.

Even before LVH develops, changes in both systolic and diastolic function can be seen.

Hypertrophy as a response to the increased afterload associated with elevated systemic vascular resistance is necessary and protective up to a certain point. Beyond that point, a variety of dysfunctions accompany LVH, including lower coronary vasodilatory capacity, depressed left ventricular wall mechanics, and abnormal left ventricular diastolic filling pattern.

It is known that treatment of arterial hypertension will cause LVH to regress. Treatment with all antihypertensive drugs except those that further activate sympathetic nervous system activity, for example, direct vasodilators such as hydralazine when used alone, has been shown to cause LVH regression. With regression, left ventricular function usually improves and cardiovascular morbidity decreases.

The various alterations of systolic and diastolic function seen with LVH obviously can progress into congestive heart failure (CHF).

A further cause for left ventricular hypertrophy may be aortic stenosis (AS).

In AS, left ventricular output is maintained by the presence of left ventricular hypertrophy, which may sustain a large pressure gradient across the aortic valve for many years without a reduction in cardiac output, left ventricular dilation, or the development of symptoms. Critical obstruction to left ventricular outflow is usually characterized by an effective aortic orifice area (calculated by the Gorlin formula) less than about 0.8 $cm^2$ in an average-sized adult. An aortic valve orifice of 1.0 to 1.5 $cm^2$ is considered moderate stenosis, and an orifice of 1.5 to 2.0 $cm^2$ is referred to as mild stenosis.

As contraction of the left ventricle becomes progressively more isometric, the left ventricular pressure pulse exhibits a rounded, rather than flattened, summit. The elevated left ventricular end-diastolic pressure, which is characteristic of severe AS, often reflects diminished compliance of the hypertrophied left ventricular wall.

Although the cardiac output at rest is within normal limits in most patients with severe AS, it often fails to rise normally during exertion. Late in the course of the disease, the cardiac output, stroke volume, and therefore the left ventricular-aortic pressure gradient all decline, whereas the mean left atrial, pulmonary capillary, pulmonary arterial, right ventricular systolic and diastolic, and right atrial pressures rise, often sequentially.

Left ventricular end-diastolic volume usually remains normal until late in the course of severe AS, but left ventricular mass increases in response to the chronic pressure overload, resulting in an increase in the mass/volume ratio.

When the aorta is constricted, left ventricular pressure rises, wall stress increases significantly, and both the extent and the velocity of shortening decline. The development of ventricular hypertrophy is one of the principal mechanisms by which the heart adapts to such an increased hemodynamic burden. The increased systolic wall stress induced by AS leads to concentric hypertrophy. The increase in left ventricular wall thickness is often sufficient to counterbalance the increased pressure, so that peak systolic wall tension returns to normal or remains normal if the obstruction develops slowly.

A further cause is hypertrophic cardiomyopathy. Cardiomyopathy is a primary disease of the heart muscle. Cardiomyopathies are divided into 3 main types: dilated, hypertrophic, and restrictive, based on the pathologic features. The term ischemic cardiomyopathy refers to the dilated, poorly contracting myocardium that sometimes occurs in patients with severe coronary artery disease (with or without areas of infarction). Although it does not describe a primary myocardial disorder, the term remains in common use.

Manifestations of cardiomyopathies are usually those of heart failure and vary depending on whether there is systolic dysfunction, diastolic dysfunction, or both. Some cardiomyopathies may also cause chest pain, syncope, or sudden death.

Evaluation typically includes ECG, echocardiography and CT scan and sometimes MRI, including stress testing.

Some patients require transvenous endomyocardial biopsy. Other tests are done as needed to determine the cause. Treatment depends on the specific type and cause of cardiomyopathy.

Hypertrophic cardiomyopathy includes a group of heart disorders in which the walls of the ventricles thicken (hypertrophy) and become stiff, even though the workload of the heart is not increased. Most cases of hypertrophic cardiomyopathy are caused by an inherited genetic defect. People experience fainting, chest pain, shortness of breath, and awareness of irregular heartbeats. In some people, the thickened muscle obstructs the flow of blood out of the heart below the aortic valve. This variation is called hypertrophic obstructive cardiomyopathy. A diagnosis based on physical examination findings can be made, but echocardiography is used to confirm the diagnosis.

Hypertrophic cardiomyopathy may be present at birth (congenital) or acquired later in life. Hypertrophic cardiomyopathy that is congenital and most cases that develop later are caused by an inherited genetic defect. Acquired hypertrophic cardiomyopathy may be caused by such disorders as acromegaly (excessive growth due to overproduction of growth hormone and a pheochromocytoma (a tumor that overproduces the hormone epinephrine). Neurofibromatosis, a hereditary disorder, may also cause hypertrophic cardiomyopathy.

Symptoms include fainting (syncope), chest pain, shortness of breath, and awareness of irregular heartbeats (palpitations) produced by an abnormal heart rhythm (arrhythmia). Fainting usually occurs during exertion.

Shortness of breath develops because fluid accumulates in the lungs. Fluid accumulates because the thickened, stiff heart resists filling with blood from the lungs and blood consequently pools in the lungs.

Because the ventricle walls thicken, the mitral valve (the valve that opens between the left atrium and the left ventricle) may be unable to close normally, resulting in leakage of a small amount of blood back into the left atrium. In some people, the thickened muscle obstructs the flow of blood out of the heart below the aortic valve. This variation is called hypertrophic obstructive cardiomyopathy.

About 4% of people with hypertrophic cardiomyopathy die each year. Death is usually sudden, presumably due to an abnormal heart rhythm. Death due to chronic heart failure is less common.

A preliminary diagnosis of left ventricular hypertrophy can usually be made based on the results of a physical examination. For example, the heart sounds heard through a stethoscope are usually characteristic. Echocardiography is the best way to confirm the diagnosis. Electrocardiography (ECG) and a chest x-ray are also helpful. Cardiac catheterization, an invasive procedure, is performed to measure pressures in the heart chambers only if surgery is being considered.

Lowbeer et al ("Serum cardiac troponin T, troponin I, plasma BNP and left ventricular mass index in professional football players "JOURNAL OF SCIENCE AND MEDICINE IN SPORT, SPORTS MEDICINE AUSTRALIA, BELCONNEN, AU, vol. 10, no. 5, 2007-08-24, pages 291-296) teaches the determination of serum levels of troponin T (TnT) and BNP in healthy professional football players.

Arteaga et al. ("Plasma amino-terminal pro-B-type natriuretic peptide quantification in hypertrophic cardiomyopathy" AMERICAN HEART JOURNAL, MOSBY- YEAR

BOOK INC, US, vol. 150, no. 6, 1 December 2005, pages 1228-1232) teaches that plasma NT-proBNP is elevated in hypertrophic cardiomyopathy (HCM) compared to normal controls.

Xu JIAN et al. ("GDF15/MIC-1 functions as a protective and antihypertrophic factor released from the myocardium in association with SMAO protein activation."CIRCULATION RESEARCH 17 FEB 2006, vol. 98, no. 3, 17, February 2006, pages 342-350) teaches that GDF15 is not expressed in the normal adult heart but is induced in response to conditions that promote hypertrophy and dilated cardiomyopathy.

Hypertrophic cardiomyopathy ("HCM") patients and pressure overload hypertrophy patients require close medical management in order to ameliorate their condition or to prevent the progression of their condition and to reduce morbidity and mortality. The medical management of patients includes the administration of drugs as well as interventions on the body of the subject.

As is clear from the foregoing, diagnosing LVH and distinguishing between the various forms thereof (athlete's heart, obstructive and non-obstructive hypertrophic cardiomyopathy and pressure overload hypertrophy) require a costly and time-consuming examination and interpretation by an experienced physician, in general a cardiologist.

In the art, diagnostic methods which allow to distinguish between healthy and pathological forms of LVH and between the different forms of LVH and which can easily be carried out and do not require time-consuming examination and interpretation by a skilled physician are not disclosed. Furthermore, there is silent on methods which can easily be carried out and which allow to decide on the medical treatment of LVH and the monitoring of the therapy are not disclosed. Neither disclosed are devices allowing to carried out such methods. Accordingly, there is a need for providing the above methods and devices.

The technical problem underlying the present invention can be seen as the provision of means and methods for complying with the aforementioned needs. The technical problem is solved by the embodiments characterized in the claims 1 to 15.

Therefore, the present invention relates to a method for diagnosing or distinguishing, in a subject having left ventricular hypertrophy, if the subject has physiological left ventricular hypertrophy or suffers from pathological left ventricular hypertrophy, the method comprising the steps of

a) determining the amounts of troponin T, of at least one BNP-type marker, in particular BNP or NT-proBNP, and of GDF-15 (Growth Differentiation Factor 15), in at least one sample of said subject,

b) comparing the thus determined amounts of the said markers as determined in step a) to suitable reference amounts, and

c) diagnosing if the subject has physiological left ventricular hypertrophy or is suffering from pathological left ventricular hypertrophy.

The method as disclosed herein may also comprise the steps of

a) determining the amounts of at least one marker selected from necrosis markers, at least one marker selected from cardiac function markers and at least one marker selected from inflammatory markers, in at least one sample of said subject,

b) diagnosing if the subject has physiological left ventricular hypertrophy or is suffering from pathological left ventricular hypertrophy by comparing the thus determined amounts of the said markers as determined in step a) to suitable reference amounts.

The above step of diagnosing, preferably, is based on the results of said comparison and depends from the results obtained.

Thus, the disclosure relates to a method for diagnosing or distinguishing, in a subject having left ventricular hypertrophy, if the subject has physiological left ventricular hypertrophy or suffers from pathological left ventricular hypertrophy based on the determination of at least one marker selected from necrosis markers, at least one marker selected from cardiac function markers and at least one marker selected from inflammatory markers, in at least one sample of said subject and the comparison of the determined amounts of said markers to reference amounts.

The disclosure also relates to the use of at least one marker selected from necrosis markers, at least one marker selected from cardiac function markers and at least one marker selected from inflammatory markers for diagnosing or distinguishing, in a subject having left ventricular hypertrophy, if the subject has physiological left ventricular hypertrophy or suffers from pathological left ventricular hypertrophy.

The proteins can be measured in one single sample or various samples of the subject, e.g. 2, 3, 4 or 5 samples. The samples may be obtained at the same time or at different time points. For example, the samples may be collected before and/or during and/or after therapy of the patient.

The cardiac function marker is a BNP-type marker, preferably NTproBNP or a variant thereof. The necrosis marker is Troponin T. The inflammatory marker is GDF-15. In a preferred embodiment of the above method, the amounts of NTproBNP, Troponin T, and GDF-15 are determined.

The method of the present invention permits to diagnose, in a straightforward and simple manner, if in a subject having left ventricular hypertrophy, the subject is healthy and has physiological left ventricular hypertrophy, or if the subject suffers from a pathological form of left ventricular hypertrophy, without having to refer to costly and time-consuming methods allowing to distinguish between the physiological and pathological form (e.g.

The term "diagnosing" as used herein means assessing, identifying, evaluating or classifying if a subject has physiological left ventricular hypertrophy or suffers from pathological left ventricular hypertrophy. The term "diagnosing" also refers to distinguishing between a physiologically healthy subject and a subject suffering from pathological left ventricular hypertrophy.

The term "left ventricular hypertrophy" as used herein relates to a thickening of the walls of the ventricles, as a result or not of a pathophysiological state of the subject, i.e. the underlying cause can be a disease or not. It is found in athlete's hearts (athletic heart syndrome) as adaptation to enhanced needs for blood supply and, and this case, does not require any treatment. Left ventricular hypertrophy is also found in patients suffering from arterial hypertension, i.e. high blood pressure being a disease requiring treatment. A further reason for the occurrence of left ventricular hypertrophy is an aortic stenosis requiring treatment by an intervention.

A cardiomyopathy is a primary disease of the heart muscle. Cardiomyopathies are divided into 3 main types: dilated, hypertrophic, and restrictive, based on the pathologic features. In the context of the present invention, only hypertrophic cardiomyopathy is relevant.

Manifestations of cardiomyopathies are usually those of heart failure and vary depending on whether there is systolic dysfunction, diastolic dysfunction, or both. Some cardiomyopathies may also cause chest pain, syncope, or sudden death.

Hypertrophic cardiomyopathy includes a group of heart disorders in which the walls of the ventricles thicken (hypertrophy) and become stiff, even though the workload of the heart is not increased. Most cases of hypertrophic cardiomyopathy are caused by an inherited genetic defect. People experience fainting, chest pain, shortness of breath, and awareness of irregular heartbeats. A diagnosis based on physical examination findings can be made, but echocardiography is used to confirm the diagnosis.

The terms "physiological", "physiologically healthy" and "physiological left ventricular hypertrophy" refer to the state of a healthy individual exhibiting left ventricular hypertrophy. The individual does not suffer from hypertrophic non-obstructive cardiomyopathy, hypertrophic obstructive cardiomyopathy, or pressure overload hypertrophy". It is found in athlete's hearts (athletic heart syndrome) as adaptation to enhanced needs for blood supply and, and this case, does not require any treatment. In the context of the present application, this state or phenomenon will be referred to as "physiological left ventricular hypertrophy" or "physiological hypertrophy". The person skilled in the art is aware that this phenomenon is generally found in athlete's hearts (athletic heart syndrome) as adaptation to enhanced needs for blood supply and is not a pathological state. It does not require any treatment.

The terms "pathological" and "pathological left ventricular hypertrophy" refer to the state of a non-healthy individual exhibiting left ventricular hypertrophy. The person skilled in the art is aware that this phenomenon is found in hypertrophic cardiomyopathy, which can be sub-divided into hypertrophic non-obstructive cardiomyopathy and hypertrophic obstructive cardiomyopathy. The person skilled in the art is also aware that the non-healthy individual can also suffer from "pressure overload hypertrophy", which can be subdivided into hypertensive left ventricular hypertrophy, which in the present application will also be referred to as "pathological hypertensive left ventricular hypertrophy", or hypertrophy caused by aortic stenosis, which in the present application will generally be referred to as "hypertrophy associated with aortic stenosis". All pathological states mentioned beforehand require treatment.

The person skilled in the art, furthermore, is aware of the pathomechanisms leading to the above-mentioned pathological states of left ventricular hypertrophy. These states include arterial hypertension, aortic stenosis and hypertrophic cardiomyopathy HCM.

As already mentioned beforehand, more than one pathomechanism may be the cause for the occurrence of pathological left ventricular hypertrophy in a subject.

In general, before the method of the present invention to distinguish between pathological and physiological left ventricular hypertrophy is carried out, left ventricular hypertrophy is diagnosed by the methods known to the person skilled in the art, in general stethoscopy and/or ECG and/or chest x-ray and/or echocardiography. In a preferred embodiment these additional diagnostic steps are part of the present invention. This may also apply for the other methods of the present invention which relate to distinguishing between different forms of left ventricular hypertrophy, methods of therapy decisions and monitoring.

The person skilled in the art is aware of methods to diagnose if the subject suffers from physiological and/or pathological left ventricular hypertrophy. In a subject which is known to have left ventricular hypertrophy, the differentiation between pathological and physiological is in general costly, time-consuming and requires medical skill and experience. Further to the methods cited above, a diagnosis is usually established on the basis of medical history, obvious signs of a cardiac dysfunction (e.g. fatigue, fainting (syncope), chest pain, shortness of breath, awareness of irregular heartbeats (palpitations) produced by an abnormal heart rhythm (arrhythmia)) and examination of vital body functions (e.g. blood pressure).

For example, a subject is diagnosed as having cardiac left ventricular hypertrophy. In such a subject, hypertensive left ventricular hypertrophy may be diagnosed by measuring blood pressure and/or medical history. Hypertrophic cardiomyopathy can be diagnosed by exclusion of other forms of hypertrophy (as hypertensive left ventricular hypertrophy) and/or by genetic examination. A known risk factor is a family history of hypertrophic cardiomyopathy or of unexplained sudden death. In patients diagnosed with hypertrophic cardiomyopathy, obstructive hypertrophic cardiomyopathy can be diagnosed by measuring the outflow gradient.

The above-referenced methods of diagnosis (distinguishing between the various form of left ventricular hypertrophy) do not only apply in respect to distinguishing between physiological and pathological left ventricular hypertrophy, but also in respect to distinguishing between the various forms of pathological left ventricular hypertrophy, which is a further object of the present invention as specified hereinafter.

The present inventors found that based on the measurement of the amount of troponin T, of at least one BNP-type marker, in particular BNP or NT-proBNP, and of GDF-15 in the sample from a subject it was possible to diagnose or distinguish in a subject having left ventricular hypertrophy, if the subject has physiological left ventricular hypertrophy or suffers from pathological left ventricular hypertrophy, as it is for example evident from the examples.

The method of the present invention, preferably, is an in vitro method. Preferably, the amounts of at least one marker is determined in a sample obtained from said subject. Moreover, it may comprise steps in addition to those explicitly mentioned above. For example, further steps may relate to sample pre-treatments or evaluation of the results obtained by the method. The method of the present invention may be also used for monitoring, confirmation, and subclassification of the subject. The method may be carried out manually or assisted by automation. Preferably, step (a), (b) and/or (c) may in total or in part be assisted by automation, e.g., by a suitable robotic and sensory equipment for the determination in step (a) or a computer-implemented calculation in step (b).

As will be understood by those skilled in the art, such an assessment is usually not intended to be correct for all (i.e. 100%) of the subjects to be identified. The term, however, requires that a statistically significant portion of subjects can be identified (e.g. a cohort in a cohort study). Whether a portion is statistically significant can be determined without further ado by the person skilled in the art using various well known statistic evaluation tools, e.g., determination of confidence intervals, p-value determination, Student's t-test, Mann-Whitney test etc.. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.1, 0.05, 0.01, 0.005, or 0.0001. More preferably, at least 60%, at least 70%, at least 80% or at least 90% of the subjects of a population can be properly identified by the method of the present invention.

The terms "individual", "subject" and "patient" may be used interchangeably herein and relate to an animal, preferably a mammal, and, more preferably, a human.

However, it is envisaged in accordance with the aforementioned method of the present invention that the subject shall be a subject having left ventricular hypertrophy, wherein the hypertrophy can be physiological or pathological. Pathological hypertrophy is caused by arterial hypertension, aortic stenosis or hypertrophic cardiomyopathy. Said subject shall exhibit symptoms and/or physical signs known to be associated with arterial hypertension, aortic stenosis or hypertrophic cardiomyopathy.

The method of the present invention makes use of so-called "markers" or "molecular markers". These terms are known to the person skilled in the art and refer to polypeptides or proteins which are expressed in the body of the subject. On the one hand, the expression or elevated expression can be the consequence of a pathophysiological state which has occurred or is occurring in the subject, and an elevated amount, in respect to "normal" values (which, as the case may be, can be zero) measured in a physiologically healthy subject, is indicative of the pathophysiological state (or the "disease") occurring in the subject. On the other hand, the protein can be expressed in certain amounts in physiologically healthy subjects, and the expression is raised in consequence of a pathophysiological state which has occurred or is occurring in the subject.

In the context of the present invention, the markers which are measured all belong to the first group, i.e. they are expressed or expressed in higher amounts than normal if the subject suffers from a pathophysiological state or disease. All the marker types and markers employed in the present invention are known to the person skilled in the art.

Necrosis markers indicate cell death having occurring in the myocard of the subject, which can occur after prolonged states of ischemia or as a consequence of apoptosis.

Cardiac function markers are indicative of a malfunction of the myocard, i.e. the muscle tissue in the myocard is weaker than normal and cannot contract as does healthy tissue, meaning that the heart has to perform harder than normal to ensure a sufficient blood supply to the body.

Inflammatory markers are indicative of inflammatory processes occurring in the body of the individual. Angiogenesis markers are indicative of angiogenetic (i.e. blood vessel forming) processes occurring in the body of the individual, as a consequence of occlusion or partial occlusion of blood vessels, in general following atherosclerosis.

Patients suffering from myocardial infarction MI can be diagnosed using cardiac troponins, preferably troponin T or I, most preferably troponin T. Myocardial infarction is regarded as being caused by a necrotic state of the myocard, i.e. cell death. Cardiac troponins are released following cell death and can hence be used for the diagnosis of MI. If the amount of Troponin T in the blood is elevated, i.e. above 0.1 ng/ml, an acute cardiovascular event is assumed and the patent is treated accordingly. However, it is known that cardiac troponins are also released (in small amounts) in pathological states preceding cell death, e.g. ischemia.

Heart failure is a condition that can result from any structural or functional cardiac disorder that impairs the ability of the heart to fill with or pump a sufficient amount of blood throughout the body. Even with the best therapy, heart failure is associated with an annual mortality of about 10 %. Heart failure is a chronic disease; it can, inter alia, occur either following an acute cardiovascular event (like myocardial infarction), or it can occur e.g. as a consequence of inflammatory or degenerative changes in myocardial tissue. Heart failure patients are classified according to the NYHA system in classes I, II, III and IV. A patient having heart failure will not be able to fully restore his health without receiving a therapeutical treatment.

Myocardial dysfunction is a general term, describing several pathological states of the heart muscle (myocard). A myocardial dysfunction may be a temporary pathological state (caused by e.g. ischemia, toxic substances, alcohol, ...), contrary to heart failure. Myocardial dysfunction may disappear after removing the underlying cause. A symptomless myocardial dysfunction may, however, also develop into heart failure (which has to be treated in a therapy). A myocardial dysfunction may, however, also be a heart failure, a chronic heart failure, even a severe chronic heart failure.

Myocardial dysfunction and heart failure often remain undiagnosed, particularly when the condition is considered "mild". The conventional diagnostic techniques for heart failure are based on the well known vascular volume stress marker NT-proBNP. Especially patients which suffer from heart failure would urgently need a supportive therapy of heart failure. On the other hand, as a consequence of an incorrect diagnosis of heart failure, many patients will receive a treatment regimen which is insufficient or which may have even adverse side effects.

The preferred cardiac function markers of the present invention are BNP-type markers, in particular BNP or NT-proBNP.

The term "natriuretic peptide" comprises Atrial Natriuretic Peptide (ANP)-type and Brain Natriuretic Peptide (BNP)-type peptides and variants thereof having the same predictive potential. Natriuretic peptides comprise ANP-type and BNP-type peptides and variants thereof (see e.g. Bonow, 1996, Circulation 93: 1946-1950). ANP-type peptides comprise pre-proANP, proANP, NT-proANP, and ANP. BNP-type peptides comprise pre-proBNP, proBNP, NT-proBNP, and BNP. The pre-pro peptide (134 amino acids in the case of pre-proBNP) comprises a short signal peptide, which is enzymatically cleaved off to release the pro peptide (108 amino acids in the case of proBNP). The pro peptide is further cleaved into an N-terminal pro peptide (NT-pro peptide, 76 amino acids in case of NT-proBNP) and the active hormone (32 amino acids in the case of BNP, 28 amino acids in the case of ANP). Preferably, natriuretic peptides according to the present invention are NT-proANP, ANP, and, more preferably, NT-proBNP, BNP, and variants thereof. ANP and BNP are the active hormones and have a shorter half-life than their respective inactive counterparts, NT-proANP and NT-proBNP. BNP is metabolised in the blood, whereas NT-proBNP circulates in the blood as an intact molecule and as such is eliminated renally. The in-vivo half-life of NTproBNP is 120 min longer than that of BNP, which is 20 min (Smith 2000, J Endocrinol. 167: 239-46.). Preanalytics are more robust with NT-proBNP allowing easy transportation of the sample to a central laboratory (Mueller 2004, Clin Chem Lab Med 42: 942-4.). Blood samples can be stored at room temperature for several days or may be mailed or shipped without recovery loss. In contrast, storage of BNP for 48 hours at room temperature or at 4° Celsius leads to a concentration loss of at least 20 % (Mueller loc.cit.; Wu 2004, Clin Chem 50: 867-73.). Therefore, depending on the time-course or properties of interest, either measurement of the active or the inactive forms of the natriuretic peptide can be advantageous. The most preferred natriuretic peptides according to the present invention are NT-proBNP or variants thereof. As briefly discussed above, the human NT-proBNP, as referred to in accordance with the present invention, is a polypeptide comprising, preferably, 76 amino acids in length corresponding to the N-terminal portion of the human NT-proBNP molecule. The structure of the human BNP and NT-proBNP has been described already in detail in the prior art, e.g., WO 02/089657, WO 02/083913 or Bonow loc. cit. Preferably, human NT-proBNP as used herein is human NT-proBNP as disclosed in EP 0 648 228 B1. The NT-proBNP referred to in accordance with the disclosure further encompasses allelic and other variants of said specific sequence for human NT-proBNP discussed above. Specifically, envisaged are variant polypeptides which are on the amino acid level preferably, at least 50%, 60%, 70%, 80%, 85%, 90%, 92%, 95%, 97%, 98%, or 99% identical to human NT-proBNP, preferably over the entire length of human NT-proBNP. The degree of identity between two amino acid sequences can be determined by algorithms well known in the art. Preferably, the degree of identity is to be determined by comparing two optimally aligned sequences over a comparison window, where the fragment of amino acid sequence in the comparison window may comprise additions or deletions (e.g., gaps or overhangs) as compared to the reference sequence (which does not comprise additions or deletions) for optimal alignment. The percentage is calculated by determining the number of positions at which the identical amino acid residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith and Waterman Add. APL. Math. 2:482 (1981), by the homology alignment algorithm of Needleman and Wunsch J. Mol. Biol. 48:443 (1970), by the search for similarity method of Pearson and Lipman Proc. Natl. Acad Sci. (USA) 85: 2444 (1988), by computerized implementations of these algorithms (GAP, BESTFIT, BLAST, PASTA, and TFASTA in the Wisconsin Genetics Software Package, Genetics Computer Group (GCG), 575 Science Dr., Madison, WI), or by visual inspection. Given that two sequences have been identified for comparison, GAP and BESTFIT are preferably employed to determine their optimal alignment and, thus, the degree of identity. Preferably, the default values of 5.00 for gap weight and 0.30 for gap weight length are used. Variants referred to above may be allelic variants or any other species specific homologs, paralogs, or orthologs. Substantially similar and also envisaged are proteolytic degradation products which are still recognized by the diagnostic means or by ligands directed against the respective full-length peptide. Also encompassed are variant polypeptides having amino acid deletions, substitutions, and/or additions compared to the amino acid sequence of human NT-proBNP as long as the said polypeptides have NT-proBNP properties. NT-proBNP properties as referred to herein are immunological and/or biological properties. Preferably, the NT-proBNP variants have immunological properties (i.e. epitope composition) comparable to those of humanNT-proBNP. Thus, the variants shall be recognizable by the aforementioned means or ligands used for determination of the amount of the natriuretic peptides. Biological and/or immunological NT-proBNP properties can be detected by the assay described in Karl et al. (Karl 1999, Scand J Clin Lab Invest 230:177-181), Yeo et al. (Yeo 2003, Clinica Chimica Acta 338:107-115). Variants also include posttranslationally modified peptides such as glycosylated peptides. Further, a variant is also a peptide or polypeptide which has been modified after collection of the sample, for example by covalent or non-covalent attachment of a label, particularly a radioactive or fluorescent label, to the peptide.

The necrosis marker employed in the present invention is cardiac troponin T.

The term "cardiac Troponin" as disclosed herein refers to all Troponin isoforms expressed in cells of the heart and, preferably, the subendocardial cells. These isoforms are well characterized in the art as described, e.g., in Anderson 1995, Circulation Research, vol. 76, no. 4: 681-686 and Ferrieres 1998, Clinical Chemistry, 44: 487-493. Preferably, cardiac Troponin refers to Troponin T and/or Troponin I, and, most preferably, to Troponin T. It is to be understood that isoforms of Troponins may be determined in the disclosed method together, i.e. simultaneously or sequentially, or individually, i.e. without determining the other isoform at all. Amino acid sequences for human Troponin T and human Troponin I are disclosed in Anderson, loc cit and Ferrieres 1998, Clinical Chemistry, 44: 487-493.

The term "cardiac Troponin" as disclosed herein encompasses also variants of the aforementioned specific Troponins, i.e., preferably, of Troponin I, and more preferably, of Troponin T. Such variants have at least the same essential biological and immunological properties as the specific cardiac Troponins. In particular, they share the same essential biological and immunological properties if they are detectable by the same specific assays referred to in this specification, e.g., by ELISA Assays using polyclonal or monoclonal antibodies specifically recognizing the said cardiac Troponins. Moreover, it is to be understood that a variant as referred to in accordance with the disclosure shall have an amino acid sequence which differs due to at least one amino acid substitution, deletion and/or addition wherein the amino acid sequence of the variant is still, preferably, at least about 50%, at least about 60%, at least about 70%, at least about 80%, at least about 85%, at least about 90%, at least about92%, at least about95%, at least about 97%, at least about 98%, or at least about 99% identical with the amino sequence of the specific Troponin. Variants may be allelic variants or any other species specific homologs, paralogs, or orthologs. Moreover, the variants referred to herein include fragments of the specific cardiac Troponins or the aforementioned types of variants as long as these fragments have the essential immunological and biological properties as referred to above. Preferably, the cardiac troponin variants have immunological properties (i.e. epitope composition) comparable to those of human troponin T or troponin I. Thus, the variants shall be recognizable by the aforementioned means or ligands used for determination of the amount of the cardiac troponins. Thus, the variants shall be recognizable by the aforementioned means or ligands used for determination of the amount of the cardiac troponins. Such fragments may be, e.g., degradation products of the Troponins. Further included are variants which differ due to posttranslational modifications such as phosphorylation or myristilation. Preferably the biological property of troponin I and its variant is the ability to inhibit actomyosin ATPase or to inhibit angiogenesis in vivo and in vitro, which may e.g. be detected based on the assay described by Moses et al. 1999 PNAS USA 96 (6): 2645-2650). Preferably the biological property of troponin T and its variant is the ability to form a complex with troponin C and I, to bind calcium ions or to bind to tropomyosin, preferably if present as a complex of troponin C, I and T or a complex formed by troponin C, troponin I and a variant of troponin T.

Preferably, the amount of a troponin T is determined with a very sensitive troponin T test system in order to allow a reliable determination of very low cardiac troponin amounts, preferably said test system is capable of determining amounts of 0.002 ng/ml troponin in a sample, preferably, in a blood, blood serum or blood plasma sample. A particularly preferred Troponin T assay is the Elecsys® 2010 analyzer (Roche Diagnostics) with a detection limit of from about 0.001 ng/ml to about 0.0015 ng/ml, in general about 0.0015 ng/ml.

The inflammatory marker of the method of the present invention is GDF-15. The term "Growth-Differentiation Factor-15" or "GDF-15" relates to a polypeptide being a member of the transforming growth factor (TGF)-β cytokine superfamily. The terms polypeptide, peptide and protein are used interchangeable throughout this specification. GDF-15 was originally cloned as macrophage-inhibitory cytokine-1 and later also identified as placental transforming growth factor-β, placental bone morphogenetic protein, non-steroidal anti-inflammatory drug-activated gene-1, and prostate-derived factor (Bootcov loc cit; Hromas, 1997 Biochim Biophys Acta 1354:40-44; Lawton 1997, Gene 203:17-26; Yokoyama-Kobayashi 1997, J Biochem (Tokyo), 122:622-626; Paralkar 1998, J Biol Chem 273:13760-13767). Similar to other TGF-β-related cytokines, GDF-15 is synthesized as an inactive precursor protein, which undergoes disulfide-linked homodimerization. Upon proteolytic cleavage of the N-terminal pro-peptide, GDF-15 is secreted as a ~28 kDa dimeric protein (Bauskin 2000, Embo J 19:2212-2220). Amino acid sequences for GDF-15 are disclosed in WO99/06445, WO00/70051, WO2005/113585, Bottner 1999, Gene 237: 105-111, Bootcov loc. cit, Tan loc. cit., Baek 2001, Mol Pharmacol 59: 901-908, Hromas loc cit, Paralkar loc cit, Morrish 1996, Placenta 17:431-441 or Yokoyama-Kobayashi loc cit.. GDF-15 as disclosed herein encompasses also variants of the aforementioned specific GDF-15 polypeptides. Such variants have at least the same essential biological and immunological properties as the specific GDF-15 polypeptides. In particular, they share the same essential biological and immunological properties if they are detectable by the same specific assays referred to in this specification, e.g., by ELISA assays using polyclonal or monoclonal antibodies specifically recognizing the said GDF-15 polypeptides. A preferred assay is described in the accompanying Examples. Moreover, it is to be understood that a variant as referred to in accordance with the disclosure shall have an amino acid sequence which differs due to at least one amino acid substitution, deletion and/or addition wherein the amino acid sequence of the variant is still, preferably, at least about 50%, at least about 60%, at least about 70%, at least about 80%, at least about 85%, at least about 90%, at least about 92%, at least about 95%, at least about 97%, at least about 98%, or at least about 99% identical with the amino sequence of the specific GDF-15 polypeptides, preferably with the amino acid sequence of human GDF-15, more preferably over the entire length of the specific GDF-15, e.g. of human GDF-15. The degree of identity between two amino acid sequences can be determined by algorithms well known in the art. Preferably, the degree of identity is to be determined by comparing two optimally aligned sequences over a comparison window, where the fragment of amino acid sequence in the comparison window may comprise additions or deletions (e.g., gaps or overhangs) as compared to the reference sequence (which does not comprise additions or deletions) for optimal alignment. The percentage is calculated by determining the number of positions at which the identical amino acid residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith and Waterman Add. APL. Math. 2:482 (1981), by the homology alignment algorithm of Needleman and Wunsch J. Mol. Biol. 48:443 (1970), by the search for similarity method of Pearson and Lipman Proc. Natl. Acad Sci. (USA) 85: 2444 (1988), by computerized implementations of these algorithms (GAP, BESTFIT, BLAST, PASTA, and TFASTA in the Wisconsin Genetics Software Package, Genetics Computer Group (GCG), 575 Science Dr., Madison, WI), or by visual inspection. Given that two sequences have been identified for comparison, GAP and BESTFIT are preferably employed to determine their optimal alignment and, thus, the degree of identity. Preferably, the default values of 5.00 for gap weight and 0.30 for gap weight length are used. Variants referred to above may be allelic variants or any other species specific homologs, paralogs, or orthologs. Moreover, the variants referred to herein include fragments of the specific GDF-15 polypeptides or the aforementioned types of variants as long as these fragments have the essential immunological and biological properties as referred to above. Such fragments may be, e.g., degradation products of the GDF-15 polypeptides. Further included are variants which differ due to posttranslational modifications such as phosphorylation or myristylation.

The preferred angionesis marker of the disclosure is P1GF or a variant thereof.

The term "reference amounts" as used herein in this embodiment of the invention refers to amounts of the polypeptides which allow allocating the left ventricular hypertrophy as being that of a healthy individual exhibiting physiological hypertrophy or that of a non-healthy individual exhibiting pathological hypertrophy.

Therefore, the reference amounts will in general be derived from a subject known to have physiological left ventricular hypertrophy, in general a subject having an athlete's heart.

The following values are indicative for a healthy individual having physiological left ventricular hypertrophy:

Necrosis marker, preferably a cardiac troponin, more preferably troponin I or troponin T, in particular troponin T referred to herein: preferably $\leq$ about 5 pg/ml, more preferably $\leq$ about 4 pg/ml, most preferably $\leq$ about 3 pg/ml. Also preferably, the necrosis marker is below the 75th percentile, preferably below the 95th percentile, of a collective of patients suffering from physiological left ventricular hypertrophy.

Cardiac function marker, preferably a natriuretic peptide, more preferably BNP or NT-proBNP, in particular NT-proBNP as referred to herein: preferably $\leq$ about 75 pg/ml, in particular $\leq$ about 50 pg/ml, most preferably $\leq$ about 20 pg/ml. Also preferably, the cardiac function marker is below the 75th percentile of a collective of individuals having physiological left ventricular hypertrophy.

Inflammatory marker, in particular GDF-15 referred to herein: preferably $\leq$ about 600 pg/ml, more preferably $\leq$ about 500 pg/ml, most preferably $\leq$ about 400 pg/ml. Also preferably, the inflammatory marker is below the 75th percentile of a collective of indivuals having physiological left ventricular hypertrophy.

The following values are indicative for a non-healthy individual having pathological left ventricular hypertrophy: troponin T: > about 5 pg/ml; BNP-type marker, preferably BNP or NT-proBNP, in particular NT-proBNP: > about 75 pg/ml; GDF-15: > about 600 pg/ml.

The above-referenced values, preferably, are those for serum samples.

The term "about" as used herein refers to +/- 20%, preferably +/-10%, preferably, +/- 5% of a given measurement or value.

In all embodiments of the present invention, the amounts/levels of the respective markers as referred to in claim 1 indicating if an individual has physiological left ventricular hypertrophy or suffers from pathological left ventricular hypertrophy or is an healthy individual, are determined by methods known to the person skilled in the art.

In general, for determining the respective amounts/levels or amount ratios allowing to establish the desired diagnosis, ("threshold", "reference amount"), the amount(s)/level(s) or amount ratios of the respective peptide or peptides are determined in appropriate patient groups. According to the diagnosis to be established, the patient group may, for example, comprise only healthy individuals, or may comprise healthy individuals and individuals suffering from the pathophysiological state which is to be determined, or may comprise only individuals suffering from the pathophysiological state which is to be determined, or may comprise individuals suffering from the various pathophysiological states to be distinguished, by the respective marker(s) using validated analytical methods. The results which are obtained are collected and analyzed by statistical methods known to the person skilled in the art. The obtained threshold values are then established in accordance with the desired probability of suffering from the disease and linked to the particular threshold value. For example, it may be useful to choose the median value, the 60th, 70th, 80th, 90th, 95th or even the 99th percentile of the healthy and/or non-healthy patient collective, in order to establish the threshold value(s), reference value(s) or amount ratios.

A reference value of a diagnostic marker can be established, and the level of the marker in a patient sample can simply be compared to the reference value. The sensitivity and specificity of a diagnostic and/or prognostic test depends on more than just the analytical "quality" of the test-they also depend on the definition of what constitutes an abnormal result. In practice, Receiver Operating Characteristic curves, or "ROC" curves, are typically calculated by plotting the value of a variable versus its relative frequency in "normal" and "disease" populations. For any particular marker, a distribution of marker levels for subjects with and without a disease will likely overlap. Under such conditions, a test does not absolutely distinguish normal from disease with 100% accuracy, and the area of overlap indicates where the test cannot distinguish normal from disease. A threshold is selected, above which (or below which, depending on how a marker changes with the disease) the test is considered to be abnormal and below which the test is considered to be normal. The area under the ROC curve is a measure of the probability that the perceived measurement will allow correct identification of a condition. ROC curves can be used even when test results don't necessarily give an accurate number. As long as one can rank results, one can create an ROC curve. For example, results of a test on "disease" samples might be ranked according to degree (say 1=low, 2=normal, and 3=high). This ranking can be correlated to results in the "normal" population, and a ROC curve created. These methods are well known in the art. See, e.g., Hanley et al, Radiology 143: 29-36 (1982).

Markers and/or marker panels are selected to exhibit at least about 70% sensitivity, more preferably at least about 80% sensitivity, even more preferably at least about 85% sensitivity, still more preferably at least about 90% sensitivity, and most preferably at least about 95% sensitivity, combined with at least about 70% specificity, more preferably at least about 80% specificity, even more preferably at least about 85% specificity, still more preferably at least about 90% specificity, and most preferably at least about 95% specificity. In particularly preferred embodiments, both the sensitivity and specificity are at least about 75%, more preferably at least about 80%, even more preferably at least about 85%, still more preferably at least about 90%, and most preferably at least about 95%. The term "about" in this context refers to +/- 5% of a given measurement.

A positive likelihood ratio, negative likelihood ratio, odds ratio, or hazard ratio is used as a measure of a test's ability to predict risk or diagnose a disease. In the case of a positive likelihood ratio, a value of 1 indicates that a positive result is equally likely among subjects in both the "diseased" and "control" groups; a value greater than 1 indicates that a positive result is more likely in the diseased group; and a value less than 1 indicates that a positive result is more likely in the control group. In the case of a negative likelihood ratio, a value of 1 indicates that a negative result is equally likely among subjects in both the "diseased" and "control" groups; a value greater than 1 indicates that a negative result is more likely in the test group; and a value less than 1 indicates that a negative result is more likely in the control group. In certain preferred embodiments, markers and/or marker panels are preferably selected to exhibit a positive or negative likelihood ratio of at least about 1.5 or more or about 0.67 or less, more preferably at least about 2 or more or about 0.5 or less, still more preferably at least about 5 or more or about 0.2 or less, even more preferably at least about 10 or more or about 0.1 or less, and most preferably at least about 20 or more or about 0.05 or less. The term "about" in this context refers to +/- 5% of a given measurement.

In the case of an odds ratio, a value of 1 indicates that a positive result is equally likely among subjects in both the "diseased" and "control" groups; a value greater than 1 indicates that a positive result is more likely in the diseased group; and a value less than 1 indicates that a positive result is more likely in the control group. In certain preferred embodiments, markers and/or marker panels are preferably selected to exhibit an odds ratio of at least about 2 or more or about 0.5 or less, more preferably at least about 3 or more or about 0.33 or less, still more preferably at least about 4 or more or about 0.25 or less, even more preferably at least about 5 or more or about 0.2 or less, and most preferably at least about 10 or more or about 0.1 or less. The term "about" in this context refers to +/- 5% of a given measurement.

In the case of a hazard ratio, a value of 1 indicates that the relative risk of an endpoint (e.g., death) is equal in both the "diseased" and "control" groups; a value greater than 1 indicates that the risk is greater in the diseased group; and a value less than 1 indicates that the risk is greater in the control group. In certain preferred embodiments, markers and/or marker panels are preferably selected to exhibit a hazard ratio of at least about 1.1 or more or about 0.91 or less, more preferably at least about 1.25 or more or about 0.8 or less, still more preferably at least about 1.5 or more or about 0.67 or less, even more preferably at least about 2 or more or about 0.5 or less, and most preferably at least about 2.5 or more or about 0.4 or less. The term "about" in this context refers to +/- 5% of a given measurement.

While exemplary panels are described herein, one or more markers may be replaced, added, or subtracted from these exemplary panels while still providing clinically useful results. Panels may comprise both specific markers of a disease (e.g., markers that are increased or decreased in bacterial infection, but not in other disease states) and/or non-specific markers (e.g., markers that are increased or decreased due to inflammation, regardless of the cause; markers that are increased or decreased due to changes in hemostasis, regardless of the cause, etc.). While certain markers may not individually be definitive in the methods described herein, a particular "fingerprint" pattern of changes may, in effect, act as a specific indicator of disease state. As discussed above, that pattern of changes may be obtained from a single sample, or may optionally consider temporal changes in one or more members of the panel (or temporal changes in a panel response value).

In order to test if a chosen reference value yields a sufficiently safe diagnosis of patients suffering from the disease of interest, one may for example determine the efficiency (E) of the disclosed methods for a given reference value using the following formula:

$$E = (TP / TO) \times 100;$$

wherein TP = true positives and TO = total number of tests = TP + FP + FN + TN, wherein FP = false positives; FN = false negatives and TN = true negatives. E has the following range of values: 0 < E < 100). Preferably, a tested reference value yields a sufficiently safe diagnosis provided the value of E is at least about 50, more preferably at least about 60, more preferably at least about 70, more preferably at least about 80, more preferably at least about 90, more preferably at least about 95, more preferably at least about 98.

The diagnosis if individuals are healthy or suffer from a certain pathophysiological state is made by established methods known to the person skilled in the art. The methods differ in respect to the individual pathophysiological state.

The algorithms to establish the desired diagnosis are laid out in the present application, in the passages referring to the respective embodiment, to which reference is made.

Accordingly, the disclosure also comprises a method of determining the threshold level indicative for a physiological and/or a pathological state and/or a certain pathological state, comprising the steps of determining in appropriate patient groups the levels of the appropriate marker(s), collecting the data and analyzing the data by statistical methods and establishing the threshold values.

In the present invention, the appropriate markers are troponin T, at least one BNP-type marker, in particular BNP or NT-proBNP, GDF-15 and, in some embodiments, P1GF. The individuals/subjects may comprise healthy individuals having physiological left ventricular hypertrophy; and/or individuals having pathological left ventricular hypertrophy; and/or individuals having obstructive left ventricular cardiomyopathy, non-obstructive left ventricular cardiomyopathy, hypertensive left ventricular hypertrophy, and/or hypertrophy associated with aortic stenosis (both latter forms being referred to as "pressure overload hypertrophy" in the present application.

For all above-cited markers the lowest values correspond to a physiological state in an absolutely healthy individual, whereas higher values may correspond to a physiological state in an individual which may have a slight health impairment, but which is still considered as being an healthy individual; still higher values are characteristic for a non-healthy individual suffering from pathological hypertrophy.

Moreover, the reference amounts, preferably, define thresholds. Suitable reference amounts or threshold amounts may be determined by the method of the present invention from a reference sample to be analyzed together, i.e. simultaneously or subsequently, with the test sample.

The person skilled in the art is aware that the values cited beforehand for troponin T and troponin I, for particular NT-proBNP, and - to a lesser extent - for GDF-15 may not apply for patients suffering from impaired renal function, preferably patients suffering from renal failure, in particular patients suffering from chronic and end stage renal failure. According to the disclosure, patients suffering from impaired renal function, preferably patients suffering from renal failure, in particular patients suffering from chronic and end stage renal failure are not comprised in (excluded from) the methods of the present invention. In another preferred embodiment, patients with renal hypertension are not comprised in (excluded from) the methods of the present invention. Preferably, the "subject having left ventricular hypertrophy" as used herein excludes patients suffering from impaired renal function, preferably patients suffering from renal failure, in particular patients suffering from chronic and end stage renal failure, more preferably patients with renal hypertension, most preferably all of the patients suffering from one of the diseases and conditions mentioned in this sentence. In this context, "renal failure" is regarded as an impaired glomerular filtration rate (GFR) lying below the usual ranges of 60 to 120 ml/min, preferably below 60 ml/min. Chronic renal failure is a long-standing, progressive deterioration of renal function which often results in end stage renal failure. End stage renal failure is diagnosed when the GFR reaches a rate of up to about 30 ml/min. GFR is determined by the creatinine clearance, which is known to the person skilled in the art. Subjects with impaired renal function show higher levels of troponin I and troponin T than those cited above, due to an impaired clearance of the peptide. The levels vary with the severity of the renal impairment.

The severity of renal impairment is divided into various grades, as displayed below.

0: ≥ 90 ml/min

1: ≥ 90 ml/min with microalbuminuria

2: ≥ 60 - < 90 ml/min

3: ≥ 30 - < 60 ml/min

4: ≥ 15 - < 30 ml/min

5: < 15 ml/min

(Source:National Kidney Foundation, as published in: Am J. Kidney Dis 39 suppl 1, 2002; Clinical Practice Guidelines for chronic kidney disease)

The present invention also encompasses a method which allows to distinguish (diagnose), in a subject suffering from pathological left ventricular hypertrophy, if the subject suffers from hypertrophic non-obstructive cardiomyopathy, hypertrophic obstructive cardiomyopathy or pressure overload hypertrophy, comprising the steps of

a) determining the amounts of troponin T, of at least one BNP-type marker, in particular BNP or NT-proBNP, and of GDF-15, in at least one sample of said subject,
b) comparing the amounts to reference amounts, and
c) distinguishing between hypertrophic non-obstructive cardiomyopathy, hypertrophic obstructive cardiomyopathy and pressure overload hypertrophy, depending on the results of step b).

The disclosure also relates to the use of at least one marker selected from necrosis markers, at least one marker selected from cardiac function markers and at least one marker selected from inflammatory markers for diagnosing or distinguishing, in a subject suffering from pathological left ventricular hypertrophy, if the subject suffers from hypertrophic non-obstructive cardiomyopathy, hypertrophic obstructive cardiomyopathy or pathological hypertensive left ventricular hypertrophy.

The amount of P1GF may be determined in a sample of the subject. In case the subject displays an amount of P1GF which is at least about 12.4 pg/ml, preferably at least about 15,0 pg/ml, in particular at least about 16.7 pg/ml, the amount is indicative that the subject suffers from obstructive left ventricular hypertrophy. Also preferably, the reference amount is the 50th or 75th percentile of a collective of patients suffering from obstructive left ventricular hypertrophy.

In one embodiment of the present invention, the ratio between BNP-type marker and GDF-15, is formed. From this ratio, it is possible to distinguish if the pathological state the subject suffers from is hypertrophic non-obstructive cardiomyopathy, on the one hand, or selected from hypertrophic obstructive cardiomyopathy and pressure overload hypertrophy, on the other hand. Whereas the ratios NT-proBNP/GDF-15 in hypertrophic obstructive cardiomyopathy and pressure overload hypertrophy are to close to allow a differentiation between the two states in a subject, the ratio is higher in patients suffering from hypertrophic non-obstructive cardiomyopathy when compared to the respective ratios of NT-proBNP/GDF-15 in hypertrophic obstructive cardiomyopathy and pressure overload hypertrophy patients.

Accordingly, the present invention provides a method for diagnosing (distinguishing), in a subject suffering from pathological left ventricular hypertrophy, if the subject suffers from hypertrophic non-obstructive cardiomyopathy, on the one hand, or from a cardiomyopathy selected from hypertrophic obstructive cardiomyopathy and pressure overload hypertrophy, on the other hand, comprising the steps of

a) determining the amounts at least one BNP-type marker, in particular BNP or NT-proBNP, and of GDF-15, in at least one sample of said subject,
b) forming the ratio between a BNP-type marker and GDF-15 as determined in step a),
c) comparing the ratio to reference ratios, and
d) distinguishing between the hypertrophic non-obstructive cardiomyopathy, on the one hand, or from a cardiomyopathy selected from hypertrophic obstructive cardiomyopathy and pressure overload hypertrophy, on the other hand, depending on the results of step c).

The method disclosed herein for diagnosing (distinguishing), in a subject suffering from pathological left ventricular hypertrophy, if the subject suffers from hypertrophic non-obstructive cardiomyopathy, on the one hand, or from a cardiomyopathy selected from hypertrophic obstructive cardiomyopathy and pressure overload hypertrophy, on the other hand, may also comprise the steps of

a) determining the amounts of at least one marker selected from cardiac function markers and at least one marker selected from inflammatory markers, in at least one sample of said subject,
b) forming the ratio between a cardiac function marker and an inflammatory marker,
c) distinguishing between hypertrophic non-obstructive cardiomyopathy, on the one hand, or from a cardiomyopathy selected from hypertrophic obstructive cardiomyopathy and pressure overload hypertrophy, by comparing the amounts to reference amounts on the other hand.

The above step of distinguishing, preferably, is based on the results of said comparison and depends from the results obtained.

The disclosure, thus, relates to a method for diagnosing (distinguishing), in a subject suffering from pathological left ventricular hypertrophy, if the subject suffers from hypertrophic non-obstructive cardiomyopathy, on the one hand, or from a cardiomyopathy selected from hypertrophic obstructive cardiomyopathy and pressure overload hypertrophy, on the other hand based on the determination of the amounts of at least one marker selected from cardiac function markers and at least one marker selected from inflammatory markers, in at least one sample of said subject, the calculation of a ratio between a cardiac function marker and an inflammatory marker and the comparison of the determined amounts to reference amounts.

According to the discloure, the reference amount is a ratio of a cardiac function marker and an inflammatory marker that has been determined in a patient or a group of patients suffering from obstructive cardiomyopathy. Or the reference amount may be the ratio of a cardiac function marker and an inflammatory marker that has been determined in a patient or a group of patients suffering from hypertrophic obstructive cardiomyopathy or pressure overload hypertrophy.

The disclosure also relates to the use of at least one marker selected from cardiac function markers and at least one marker selected from inflammatory markers for the preparation of a diagnostic for distinguishing, in a subject suffering from pathological left ventricular hypertrophy, if the subject suffers from hypertrophic non-obstructive cardiomyopathy, on the one hand, or from a cardiomyopathy selected from hypertrophic obstructive cardiomyopathy and pathological hypertensive left ventricular hypertrophy, on the other hand.

The cardiac function marker is a BNP-type marker, more preferably BNP or NT-proBNP, in particular NT-proBNP. The inflammatory marker is GDF-15.

NT-proBNP/GDF-15 values indicative for the occurrence of hypertrophic non-obstructive cardiomyopathy are values $\geq$ (higher than or equal to) about 0.43, preferably $\geq$ about 0.6, in particular about $\geq$ 0.8.

NT-proBNP/GDF-15 values indicative for the occurrence of hypertrophic obstructive cardiomyopathy and pressure overload hypertrophy are values < (lower than) about 0.43, preferably < about 0.3, in particular < about 0.2.

In one further embodiment of the present invention, allowing to distinguish between the various forms of pathological left ventricular hypertrophy as laid out beforehand, the amount of P1GF is determined in a sample of the subject. In case the subject displays an amount of P1GF which is at least about 12.4 pg/ml, preferably at least about 15,0 pg/ml, in particular at least about 16.7 pg/ml, the amount is indicative that the subject suffers from in particular obstructive left ventricular hypertrophy as a subject suffering thereof shows the highest amounts of this peptide, in general around 15 pg/ml. Therefore, the reference amount is, also preferably, the $50^{th}$ or $75^{th}$ percentile of a collective of patients suffering from obstructive left ventricular hypertrophy.

In a further embodiment of the present invention, the ratio between troponin T and the GDF-15 is formed. From this ratio, it is possible to distinguish if the pathological state the subject suffers from is hypertrophic obstructive cardiomyopathy, hypertrophic non-obstructive cardiomyopathy or pressure overload hypertrophy.

Accordingly, the present invention provides a method for diagnosing (distinguishing) in a subject suffering from pathological left ventricular hypertrophy, if the subject suffers from hypertrophic non-obstructive cardiomyopathy, hypertrophic obstructive cardiomyopathy or pressure overload hypertrophy, comprising the steps of

a) determining the amounts of troponin T, and of GDF-15, in at least one sample of said subject,
b) forming the ratio between troponin T and GDF-15 as determined in step a)the necrosis marker and an inflammatory marker,
c) comparing the ratio to reference ratios, and
d) distinguishing between hypertrophic non-obstructive cardiomyopathy, hypertrophic obstructive cardiomyopathy and pressure overload hypertrophy, depending on the results of step c).

The disclosed method for diagnosing (distinguishing) in a subject suffering from pathological left ventricular hypertrophy, if the subject suffers from hypertrophic non-obstructive cardiomyopathy, hypertrophic obstructive cardiomyopathy or pressure overload hypertrophy, may also comprise the steps of

a) determining the amounts of at least one marker selected from necrosis markers and at least one marker selected from inflammatory markers, in at least one sample of said subject,
b) forming the ratio between the necrosis marker and an inflammatory marker,
c) distinguishing between hypertrophic non-obstructive cardiomyopathy, hypertrophic obstructive cardiomyopathy and pressure overload hypertrophy, by comparing the amounts to reference amounts.

The above step of distinguishing, preferably, is based on the results of said comparison and depends from the results obtained.

The present disclosure, thus, relates to a method for diagnosing (distinguishing), in a subject suffering from pathological left ventricular hypertrophy, if the subject suffers from hypertrophic non-obstructive cardiomyopathy, hypertrophic obstructive cardiomyopathy or pressure overload hypertrophy based on the determination of the amounts of at least one marker selected from necrosis markers and at least one marker selected from inflammatory markers, in at least one sample of said subject, the calculation of a ratio between the necrosis marker and the inflammatory marker and the comparison of the determined amounts to reference amounts.

The reference amount may be a ratio of a necrosis marker and an inflammatory marker that has been determined in a patient or a group of patients suffering from hypertrophic non-obstructive cardiomyopathy. Or the reference amount may be the ratio of a cardiac function marker and an inflammatory marker that has been determined in a patient or a group of patients suffering from hypertrophic obstructive cardiomyopathy. Or the reference amount may be the ratio of a cardiac function marker and an inflammatory marker that has been determined in a patient or a group of patients suffering from pressure overload hypertrophy.

The disclosure also relates to the use of at least one marker selected from necrosis markers and at least one marker selected from inflammatory markers for the preparation of a diagnostic for distinguishing, in a subject suffering from pathological left ventricular hypertrophy, if the subject suffers from hypertrophic non-obstructive cardiomyopathy, hypertrophic obstructive cardiomyopathy or pathological hypertensive left ventricular hypertrophy.

The necrosis marker is troponin T. The inflammatory marker is GDF-15.

Troponin T/GDF-15 values indicative for the occurrence of hypertrophic non-obstructive cardiomyopathy are values ≥ (higher than or equal to) about 0.01, preferably ≥ about 0.03, in particular ≥ about 0.06.

Troponin T/GDF-15 values indicative for the occurrence of hypertrophic obstructive cardiomyopathy are values ≤ (lower than or equal to) about 0.004, preferably ≤ about 0.002, in particular ≤ about 0.001.

Troponin T/GDF-15 values indicative for the occurrence of pressure overload hypertrophy are values ranging from > about 0.004 to < about 0.01, preferably about 0.006, in particular about 0.008.

In one further embodiment of the present invention, allowing to distinguish between the various forms of pathological left ventricular hypertrophy as laid out beforehand, the amount of P1GF is determined in a sample of the subject. By this, it is possible to recognize in particular obstructive left ventricular hypertrophy as a subject suffering thereof shows the high amounts of this peptide.

Accordingly, the present invention furthermore comprises a method of diagnosing hypertrophic obstructive cardiomyopathy in an individual, the method comprising determining the amount of P1GF in a sample of the subject, wherein an elevated amount of P1GF is indicative for hypertrophic obstructive cardiomyopathy.

P1GF values indicative for the occurrence of obstructive left ventricular hypertrophy are values ≥ (higher than or equal to) about 12.4 pg/ml, preferably ≥ about 15,0 pg/ml, in particular ≥ about 16.7 pg/ml. Also preferably, the reference amount is the 50th or 75th percentile of a collective of patients suffering from obstructive left ventricular hypertrophy.

The term "diagnosing" as used herein means assessing, identifying, evaluating or classifying if a subject has hypertrophic non-obstructive cardiomyopathy, hypertrophic obstructive cardiomyopathy and/or pressure overload hypertrophy (i.e. pathological hypertensive left ventricular hypertrophy or hypertrophy associated with aortic stenosis), according to the respective embodiment of the present invention. The term "diagnosing" also refers to distinguishing between the above-cited forms of pathological left ventricular hypertrophy, according to the respective embodiment of the present invention.

The person skilled in the art is aware of methods to diagnose if the subject suffers from physiological or pathological left ventricular hypertrophy. In a subject which is known to have left ventricular hypertrophy, distinguishing between pathological and physiological and, in a subject known to suffer from pathological left ventricular hypertrophy, and distinguishing between the various forms of left ventricular hypertrophy is in general costly, time-consuming and requires medical skill and experience. Methods of evaluation are known to the person skilled in the art and are typically based on medical history, obvious signs of a cardiac dysfunction (e.g. fatigue, fainting (syncope), chest pain, shortness of breath, awareness of irregular heartbeats (palpitations) produced by an abnormal heart rhythm (arrhythmia)), examination of vital body functions (e.g. blood pressure). Further evaluation includes examination using diagnostic apparatusses/devices (cardiac auscultation (stethoscopy), ECG, echocardiography, chest x-ray, radionuclide imaging, ventriculography, CT scan, MRI and/or stress testing, coronary angiography, ultrasonography). Some patients require transvenous endomyocardial biopsy. Other tests may be done as needed to determine the cause. Treatment depends on the specific type and cause of cardiomyopathy.

In general, a subject is diagnosed (e.g. by echocardiography) for left ventricular hypertrophy, if the subject's medical history indicates a certain probability, or in the course of a routine examination, or a combination of both. Prior to the diagnosis, the occurrence of LVH is often suspected, due to hints in the ECG. For example, the subject may be a competitive endurance athlete with a probability for the occurrence of left ventricular hypertrophy; or the individual may have a family history of hypertrophic cardiomyopathy or cases of sudden cardiac death.

It is then evaluated if the left ventricular hypertrophy is physiological or pathological and, as the case may be, which form of pathological left ventricular hypertrophy is present. This evaluation/diagnosis may include various examinations, diagnostic conclusions and exclusions, as laid out hereinafter.

In case of LVH in a competitive athlete, it should preferably be diagnosed if the LVH is physiological or pathological (in particular if the subject has a family history of hypertrophic cardiomyopathy or of sudden cardiac death) by a genetic examination; the genetic examination shows if there is a family history physiological or the pathological form is present and which form can be excluded. In case a left ventricular hypertrophy is diagnosed in a subject other than in a competitive athlete, left ventricular hypertrophy is pathological and the physiological form can be excluded. In case the subject has a history of hypertension, and the hypertension still persists, the subject suffers from hypertensive LVH; accordingly other forms can generally be excluded. Aortic stenosis can be diagnosed by cardiac auscultation, and determination of the left ventricular outflow gradient; aortic stenosis is often confirmed by echocardiography; this way, the other forms of pathological LVH including hypertensive LVH can be ruled out. Hypertrophic cardiomyopathy can be diagnosed by genetic examination, often after exclusion of the other forms of pathological LVH. In individuals suffering from hypertrophic cardiomyopathy, obstruction can be diagnosed by determining the left ventricular outflow gradient.

To further illustrate the invention, a subject is for example diagnosed as having cardiac hypertrophy. In such a subject, hypertensive left ventricular hypertrophy is diagnosed by measuring blood pressure and/or referring to medical history. Hypertrophic cardiomyopathy can be diagnosed by exclusion of other forms of hypertrophy (as hypertensive left ventricular hypertrophy) and/or by genetic examination. A known risk factor is a family history of hypertrophic cardiomyopathy or of unexplained sudden death. In patients diagnosed with hypertrophic cardiomyopathy, obstructive hypertrophic cardiomyopathy can be diagnosed by measuring the outflow gradient.

The diagnostic methods listed above can be used supplementary/complementary with the disclosed methods based on the determination of the markers cited in the claims.

For example, it may immediately be distinguished between physiological and pathological individuals by determining the amounts of the markers as set forth in the claims. A further example includes forming the ratio between a cardiac function marker and an inflammatory marker and diagnosing if the subject suffers from hypertrophic non-obstructive cardiomyopathy, on the one hand, or from a cardiomyopathy selected from hypertrophic obstructive cardiomyopathy and pressure overload hypertrophy, on the other hand. If the subject does not suffer from non-obstructive hypertrophic cardiomyopathy, it can be excluded and the differentiation between the remaining states of LVH can be made by one or more of the methods laid out above.

According to the disclosure, the supplementary/complementary methods as laid out beforehand can be used for the methods of deciding on the treatment of a subject as referred to above, based on the aforementioned steps. These methods are laid out hereinafter and allow to decide which pharmaceutical or pharmaceuticals should be taken by said subject or which other therapy the subject should undergo.

The markers (peptides) which are described herein can also be used for the confirmation of a diagnosis established by a conventional diagnostic method known in the art. Thus, disclosed is a method of confirming a diagnosis which is not or only partly-based on the determination of the markers used in the present invention, by determining the amounts of the markers used in the present invention, comparing these to reference amounts, and confirming or not confirming the diagnosis obtained by methods according to the state of the art.

Angiogenesis is known as the formation of new blood vessels from already existing blood vessels by a capillary sprouting process. The process is under physiological conditions essentially driven by angiogenic growth factors such as the vascular endothelial growth factor (VEGF). The expression of such angiogenic growth factors is regulated pivotally by hypoxia. Thus, if a tissue becomes ischemic, the cells will start to produce angiogenic growth factors which will attract new blood vessels to the tissue by angiogenesis.

However, the capability of a subject for angiogenesis, i.e. its angiogenic status, is dependent on complex biological parameters. Various angiogenesis promoting factors as well as inhibitors of angiogenesis have been reported (Nyberg 2005, Cancer Res 65:3967-3979).

Angiogenesis is observed during tumor growth where the growing tumor becomes more and more affected by hypoxia.

Other disease conditions which are accompanied by hypoxia and ischemia include the coronary artery diseases. Said diseases are characterized by stenosis or occlusion of vessels of the coronary artery system, e.g. by atherosclerosis or thromboembolic occlusions. Coronary artery diseases result in ischemia of the myocardium. Said ischemia, if left untreated, may severely interfere with the physiological function of the heart and result in cardiac disorders including heart failure or even myocardial infarction. For patients suffering from coronary artery diseases, an angiogenic therapy may assist in avoiding the aforementioned life-threatening conditions. Moreover, angiogenic therapies may even help to circumvent complicated cardiac interventions such as stent implantation or bypass surgery.

As set forth above already, various factors besides VEGF have been reported to play a role in angiogenesis. Placental growth factor (P1GF) is a closely related growth factor suggested to play a role in the related process of arteriogenesis together with its putative receptor Flt-1 (Khurana 2005, Circulation 111:2828-2836). Other factors which are possibly involved in arteriogenesis and angiogenesis are the members of the Transforming growth factor-beta superfamily as well as their receptors or binding partners such as the ALK receptors or Endoglin (van Laake 2006, Circulation, 114:2288-2297; Bobik 2006, Arterioscler Thromb Vasc Biol 26: 1712-1720; Bertolino 2005, Chest Supplement 128: 585-590). Fibroblast growth factor (FGF), Platlet derived growth factor (PDGF) as well as cytokines and matrix-metalloproteinases have been also described as potent angiogenic factors (Nyberg, loc.cit.).

The term "P1GF (Placental Growth Factor)" as used herein refers to a placenta derived growth factor which is a 149-amino-acid-long polypeptide and is highly homologous (53% identity) to the platelet-derived growth factor-like region of human vascular endothelial growth factor (VEGF). Like VEGF, P1GF has angiogenic activity in vitro and in vivo. For example, biochemical and functional characterization of P1GF derived from transfected COS-1 cells revealed that it is a glycosylated dimeric secreted protein able to stimulate endothelial cell growth in vitro (Maqlione1993, Oncogene 8(4):925-31. Preferably, P1GF refers to human P1GF, more preferably, to human P1GF having an amino acid sequence as shown in Genebank accession number P49763, GI: 17380553 (Genebank is available from the NCBI, USA, e.g.under www.ncbi.nlm.nih.gov/entrez). Moreover, it is to be understood that a variant as referred to in accordance with the disclosure shall have an amino acid sequence which differs due to at least one amino acid substitution, deletion and/or addition wherein the amino acid sequence of the variant is still, preferably, at least about 50%, at least about 60%, at least about 70%, at least about 80%, at least about 85%, at least about 90%, at least about 92%, at least about 95%, at least about 97%, at least about 98%, or at least about 99% identical with the amino sequence of the specific P1GF, preferably with the amino acid sequence of human P1GF, more preferably identical with the amino sequence of human P1GF over the entire length of human P1GF. Variants may be allelic variants, splice variants or any other species specific homologs, paralogs, or orthologs. Moreover, the variants referred to herein include fragments of the specific P1GF or the aforementioned types of variants as long as these fragments have the essential immunological and biological properties as referred to above. Such fragments may be, e.g., degradation products of P1GF. Further included are variants which differ due to posttranslational modifications such as phosphorylation or myristylation.

In general, before the method of the present invention to distinguish between pathological and physiological left ventricular hypertrophy is carried out, left ventricular hypertrophy is diagnosed by the methods known to the person skilled in the art, in general stethoscopy and/or ECG and/or chest x-ray and/or echocardiography. In a preferred embodiment these additional diagnostic steps are part of the present invention. This may also apply for the other methods of the present invention which relate to distinguishing between different forms of left ventricular hypertrophy, methods of therapy decisions and monitoring.

The person skilled in the art is aware of methods to distinguish between the various forms of pathological hypertrophy cited beforehand. In a subject which is known to have pathological left ventricular hypertrophy, the differentiation between the various forms is in general costly, time-consuming and requires medical skill and experience. Further to the methods cited above, a diagnosis is established on the basis of medical history, obvious signs of a cardiac dysfunction (e.g. fatigue, fainting (syncope), chest pain, shortness of breath, awareness of irregular heartbeats (palpitations) produced by an abnormal heart rhythm (arrhythmia)) and examination of vital body functions (e.g. blood pressure).

The present inventors found that based on the measurement of the amount of the markers as referred to in the claims the sample from a subject it was possible to distinguish, in a subject suffering from pathological left ventricular hypertrophy, between the various forms thereof (i.e. hypertrophic non-obstructive cardiomyopathy, hypertrophic obstructive cardiomyopathy or pressure overload hypertrophy), as it is e.g. evident from the examples.

Left ventricular hypertrophy and hypertrophic cardiomyopathy can be accompanied by more or less severe forms of myocardial dysfunction, left ventricular dysfunction or heart failure. Both terms are known to the person skilled in the art.

The disclosure therefore also relates to cardiac disorders, preferably from the group myocardial dysfunction, left ventricular dysfunction and heart failure.

The term "myocardial dysfunction" as used herein is a general term and relates to several pathological states of the myocard. A myocardial dysfunction may be a temporary pathological state (caused by e.g. ischemia, toxic substances, alcohol, ...). Myocardial dysfunction may disappear after removing the underlying cause. The myocardial dysfunction can be a symptomless myocardial dysfunction. A myocardial dysfunction, in particular a symptomless myocardial dysfunction, may also develop into heart failure. A myocardial dysfunction may also be a severe chronic heart failure. In general, a myocardial dysfunction is an impaired systolic and/or diastolic function of the heart, and a myocardial dysfunction may occur with or without heart failure. Any heart failure mentioned beforehand may be symptomless.

The term "heart failure" as used herein relates to an impaired systolic and/or diastolic function of the heart. Preferably, heart failure referred to herein is also chronic heart failure. Heart failure can be classified into a functional classification system according to the New York Heart Association (NYHA). Patients of NYHA Class I have no obvious symptoms of cardiovascular disease but already have objective evidence of functional impairment. Physical activity is not limited, and ordinary physical activity does not cause undue fatigue, palpitation, or dyspnea (shortness of breath). Patients of NYHA class II have slight limitation of physical activity. They are comfortable at rest, but ordinary physical activity results in fatigue, palpitation, or dyspnea. Patients of NYHA class III show a marked limitation of physical activity. They are comfortable at rest, but less than ordinary activity causes fatigue, palpitation, or dyspnea. Patients of NYHA class IV are unable to carry out any physical activity without discomfort. They show symptoms of cardiac insufficiency at rest. Heart failure, i.e., an impaired systolic and/or diastolic function of the heart, can be determined also by, for example, echocardiography, angiography, szintigraphy, or magnetic resonance imaging. This functional impairment can be accompanied by symptoms of heart failure as outlined above (NYHA class II-IV), although some patients may present without significant symptoms (NYHA I). Moreover, heart failure is also apparent by a reduced left ventricular ejection fraction (LVEF). More preferably, heart failure as used herein is accompanied by a left ventricular ejection fraction (LVEF) of less than 60 %, of 40 % to 60 % or of less than 40 %.

Also, in accordance with the disclosure, and with respect to the reference values cited beforehand, an increased amount of GDF-15 is indicative for inflammatory processes occurring in the body of the patient, preferably in the myocard, whereas with respect to the reference values a decreased amount of GDF-15 is indicative for the absence of inflammatory processes. Thus, in a preferred embodiment of the method of the present invention, an increased amount of GDF-15 is indicative for inflammatory processes, whereas a decreased amount of GDF-15 is indicative for the absence of inflammatory processes.

Moreover, it has been found that each of said biomarkers is statistically independent from each other.

The present invention, preferably, also relates to a method of deciding on the treatment of a subject as referred to in claim 8. Therefore, the method of the present invention allows to decide which pharmaceutical or pharmaceuticals should be taken by said subject or which other therapy the subject should undergo, e.g. to focus more on the treatment of pathological left ventricular hypertrophy (caused by one or more of arterial hypertension, aortic stenosis or hypertrophic cardiomyopathy), or to focus more on preventing further deterioration of the pathological left ventricular hypertrophy.

The present invention therefore also relates to a method of deciding on the therapy for treating pathological left ventricular hypertrophy in an individual suffering from the said disease, comprising the steps of

a) determining the amounts of troponin T, of at least one BNP-type marker, in particular BNP or NT-proBNP, and of GDF-15, in at least one sample of said subject,
b) comparing the thus determined amounts of the said markers as determined in step a) to suitable reference amounts, and
c) deciding on the therapy, based on the comparison carried out in step b).

The present invention, thus, relates to a method of deciding on the therapy for treating pathological left ventricular hypertrophy in an individual suffering from the said disease based on the determination of the amounts of the aforementioned markers, in at least one sample of said subject and the comparison of the determined amounts of the said markers to suitable reference amounts.

The disclosure also encompasses the use of at least one marker selected from necrosis markers, at least one marker selected from cardiac function markers and at least one marker selected from inflammatory markers for the preparation of a diagnostic for deciding on the therapy for treating pathological left ventricular hypertrophy in an individual suffering from the said disease.

The cardiac function marker is a BNP-type marker, more preferably BNP or NT-proBNP, in particular NT-proBNP. The inflammatory marker is GDF-15. The necrosis marker is troponin T. More preferably, the following markers are determined in combination: NT-proBNP, GDF-15 and troponin T, and optionally also P1GF.

In one embodiment of this aspect of the present invention, the angiogenesis marker P1GF is measured in addition to the at least three markers specified beforehand. By this, additional information on the appropriate therapy may be available.

In accordance with the present invention, and with respect to the above-cited reference values, a decreased amount of P1GF is indicative for an anti-angiogenic status, whereas with respect to the reference values, an increased amount of P1GF is indicative for a pro-angiogenic status. An angiogenic ("pro-angiogenic") status is indicative for the occurrence of ischemic states or processes, whereas an anti-angiogenic status is indicative for the non-occurrence of ischemic states or processes.

The supplementary/complementary methods as laid out beforehand can be used for the methods of deciding on the treatment of a subject as referred to above, based on the aforementioned steps.

The term "therapy" as used in the context of the present invention encompasses interventions on the body as well as administration of appropriate drugs for the treatment of left ventricular hypertrophy, in particular left ventricular hypertrophy caused by one or more of arterial hypertension, aortic stenosis or hypertrophic cardiomyopathy. Pharmaceuticals suitable for the treatment of left ventricular hypertrophy are well known in the art, see e.g. Heart Disease, 2005, 7th Edition, Eds. Braunwald, Elsevier Sounders, see tables 23-1, 23-6, 23-7, 23-8, 23-9, 23-10. Preferably, the administration of such pharmaceuticals aims to treat the symptoms and signs of left ventricular hypertrophy caused by one or more of arterial hypertension, aortic stenosis or hypertrophic cardiomyopathy and which aim to prevent a further progression of left ventricular hypertrophy. Accordingly, also contemplated are pharmaceuticals that aim to treat left ventricular dysfunction and/or heart failure, anti-inflammatory drugs.

The therapy may also include interventions. One preferred intervention in the context of the present invention is controlled destruction of areas of the heart muscle by alcohol (ablation), in particular TASH (transcoronary ablation of septum hypertrophy) or PTSMA (percutaneous transluminal septal myocardial ablation), in case of an obstructive hypertrophic cardiomyopathy.

Another preferred intervention is aortic valve replacement (AVR) in case of an aortic stenosis.

The disclosure includes a method of deciding on the treatment of a patient suffering from hypertrophic cardiomyopathy. The term "deciding" as used herein means assessing as to whether a certain medication or treatment should be administered to a subject having undergone the test according to the disclosure. The treatment is selected from the following:

Treatment A):

a) change in life style, in particular low sodium intake

b) administration of an agent or agents effecting cardiac function, preferably: beta blockers like proprenolol, metoprolol, bisoprolol, carvedilol, bucindolol, nebivolol; nitrates; adrenergic agonists, like dobutamine, dopamine, epinephrine, isoprotenerol, norepinephrine, phenylephrine; positive inotropic agents, like digoxin, digitoxin; diuretics, in particular loop diuretics, thiazide and thiazide-like diuretics, K-sparing diuretics, type I mineralocorticoid receptor antagonists, carbonic anhydrase inhibitors, vasopressure antagonists.

The information whether these agents should be administered is provided if an elevated level of the cardiac function marker, preferably a natriuretic peptide is measured. Suitable natriuretic peptides are BNP, NT-proBNP, ANP, NT-proANP; preferably BNP or NT-proBNP, in particular NT-proBNP.

When a level of natriuretic peptide of, in the case of NT-proBNP, $\geq$ about 300 pg/ml, preferably $\geq$ about 500 pg/ml, more preferably $\geq$ about 800 pg/ml, still more preferably $\geq$ about 2000 pg/ml is reached, one or more of the above-cited drugs should be administered. Also preferably, the reference amount is the amount of the natriuretic peptide corresponding to the 50th or 75th percentile determined in a collective of patients suffering from hypertrophic cardiomyopathy.

The levels of natriuretic peptides, in particular NT-proBNP cited beforehand ($\geq$about 300 pg/ml, preferably $\geq$ about 500 pg/ml, more preferably $\geq$ about 800 pg/ml, still more preferably $\geq$ about 2000 pg/ml) may also indicate that, in case of an obstructive hypertrophic cardiomyopathy, an ablation, in particular TASH, should be carried out The diagnosis of obstructive hypertrophic cardiomyopathy as such has to be established by methods known to the person skilled in the art, like heart sounds, echocardiography, electrocardiography (ECG), chest x-ray and/or cardiac catheterization; or by methods disclosed in the present application, like forming the ratio between a cardiac function marker (NT-proBNP) and an inflammatory marker (GDF-15).

Treatment B):

Administration of one or more anti-inflammatory drugs, preferably: ACE inhibitors, in particular Enalapril, Captopril, Ramipril, Trandolapril; angiotensin receptor antagonists and aldosterone antagonists, in particular Losartan, Valsartan, Irbesartan, Candesartan, Telmisartan, Eprosartan, Spironolactone; statines, in particular Atorvastatin, Fluvastatin, Lovastatin, Pravastatin, Rosuvastatin, Simvastatin;

The information whether these agents should be administered is provided if an elevated level of an inflammatory maker, preferrably GDF-15 which is indicative for inflammatory processes is measured.

When a level of GDF-15 of $\geq$ about 800 pg/ml, preferably $\geq$ about 1200 pg/ml, more preferably $\geq$ about 1500 pg/ml, in particular $\geq$ about 2000 pg/ml is reached, one or more of the above-cited drugs should be administered. Also preferably, the reference amount is the amount of GDF-15 corresponding to the 50th or 75th percentile determined in a collective of patients suffering from hypertrophic cardiomyopathy.

**Treatment C):**

Treatment of percutane coronary intervention: In general, the level of a necrosis marker, preferably Troponin I and/or T, in particular Troponin T, is indicative of an existing myocardial necrosis and the extent of the necrosis; in case no drop in the level of Troponin T or Troponin I is observed, then this peptide indicates heart failure and/or vascular stenosis; vascular stenosis can be treated by percutane coronary intervention.

The information whether these agents should be administered if an elevated level of Troponin I and/or Troponin T, in particular Troponin T of $\geq$ about 3 pg/ml, preferably $\geq$ about 4 pg/ml, in particular $\geq$ about 5 pg/ml which is indicative for heart failure or vascular stenosis is measured. Also preferably, the reference amount is the amount of Troponin I or Troponin T corresponding to the 50th or 75th percentile determined in a collective of patients suffering from hypertrophic cardiomyopathy.

Treatment D):

Administration of at least one medicament for a pro-angiogenic therapy

The term "pro-angiogenic therapy" as recited above relates to a therapy which induces or enhances the process of angiogenesis systemically or topically in a subject and includes the treatment of micro- and macroangiopathy. Preferably, said pro-angiogenic therapy comprises administration of a pro-angiogenic drug, preferably, selected from the group consisting of: VEGF, P1GF, Endoglin, anti-Flt-1 antibodies and ALK5 modifiers. These drugs can be used for the treatment of both micro- and macroangiopathy.

The term "susceptible" as used herein means that a statistically significant portion of subjects identified by the method as being susceptible respond to the envisaged therapy by showing angiogenesis in the affected areas of the heart.

The information whether these agents should be administered is provided if an elevated (lowered) level of P1GF which is indicative for anti-angiogenic processes is measured.

According to the aforementioned method, an increased amount of PLGF identifies a subject as being susceptible to a pro-angiogenic therapy.

When a level of P1GF of $\geq$ about 8 pg/ml, preferably $\geq$ about 10 pg/ml, more preferably $\geq$ about 12 pg/ml, in particular $\geq$ about 15 pg/ml is reached, one or more of the above-cited drugs should be administered. Also preferably, the reference amount is the amount of P1GF corresponding to the 50th or 75th percentile determined in a collective of patients suffering from hypertrophic cardiomyopathy.

The present invention furthermore relates to a method of monitoring the therapy of treating pathological left ventricular hypertrophy in an individual suffering from the said disease, comprising the steps of

a) determining the amounts of troponin T, of at least one BNP-type marker, in particular BNP or NT-proBNP, and of GDF-15 in at least one sample of said subject,
b) comparing the thus determined amounts of the said markers as determined in step a) to suitable reference amounts, and
c) as the case may be, adapting or discontinuing the therapy, based on the comparison carried out in step b).

The reference amounts cited in step b) may be the reference amounts cited in the present application in respect to the therapy decision beforehand under A), B), C) and D), or may be amounts determined before the therapy was initiated, or both.

In respect to Troponin T, a deviation of about 10%, about 20%, about 30%, about 40% or about 50%, in particular about 20%, from the corresponding reference amount is indicative for an amelioration (in case of a decrease of the marker amounts) or a deterioration (in case of an increase of the marker amounts) of the pathological state of the individual.

In respect to the BNP-type marker, preferably BNP or NT-proBNP, in particular NT-proBNP, a deviation of about 10%, about 20%, about 30%, about 40% or about 50%, in particular about 20%, from the corresponding reference amount is indicative for an amelioration (in case of a decrease of the marker amounts) or a deterioration (in case of an increase of the marker amounts) of the pathological state of the individual.

The BNP-type marker preferably BNP or NT-proBNP, in particular NT-proBNP, serves as a marker to monitor TASH therapy in an individual. It could be established, by the inventors of the present application that the level of said marker significantly diminishes after successful TASH intervention. A deviation of about 10%, about 20%, about 30%, about 40% or about 50%, in particular about 20%, from the corresponding reference amount is indicative of a successful TASH intervention.

In respect to GDF-15 a deviation of about 10%, about 20%, about 30%, about 40% or about 50%, in particular about 20%, from the corresponding reference amount is indicative for an amelioration (in case of a decrease of the marker amounts) or a deterioration (in case of an increase of the marker amounts) of the pathological state of the individual.

The present inventors found that based on the measurement of the amount of the markers as referred on in claim 10 in the sample from a subject it was possible to decide on the treatment of the various forms of pathological left ventricular hypertrophy, (i.e. hypertrophic non-obstructive cardiomyopathy, hypertrophic obstructive cardiomyopathy or pressure overload hypertrophy), and to monitor the treatment, without having to refer to costly and time-consuming diagnostic methods known to the person skilled in the art and laid out elsewhere in the specification.

In one embodiment of the present invention, the angiogenesis marker P1GF is measured in addition to the at least three markers specified beforehand. By this, additional on the pathological state of the individual and the therapy success may be available.

In respect to P1GF, a deviation of about 10%, about 20%, about 30%, about 40% or about 50%, in particular about 20%, from the corresponding reference amount is indicative for an amelioration (in case of a decrease of the marker amounts) or a deterioration (in case of an increase of the marker amounts) of the pathological state of the individual.

In general, prior to carrying out the monitoring method of the present invention, the method of deciding on the therapy of treating left ventricular hypertrophy is carried out, in a preferred embodiment of the present invention.

Determining the amount of the markers referred to in the claims relates to measuring the amount or concentration, preferably semi-quantitatively or quantitatively. Measuring can be done directly or indirectly. Direct measuring relates to measuring the amount or concentration of the peptide or polypeptide based on a signal which is obtained from the peptide or polypeptide itself and the intensity of which directly correlates with the number of molecules of the peptide present in the sample. Such a signal - sometimes referred to herein as intensity signal -may be obtained, e.g., by measuring an intensity value of a specific physical or chemical property of the peptide or polypeptide. Indirect measuring includes measuring of a signal obtained from a secondary component (i.e. a component not being the peptide or polypeptide itself) or a biological read out system, e.g., measurable cellular responses, ligands, labels, or enzymatic reaction products.

The term "sample" refers to a sample of a body fluid, to a sample of separated cells or to a sample from a tissue or an organ. Samples of body fluids can be obtained by well known techniques and include, preferably, samples of blood, plasma, serum, urine, samples of blood, plasma or serum. It is to be understood that the sample depends on the marker to be determined. Therefore, it is encompassed that the polypeptides as referred to herein are determined in different samples. Cardiac troponins and natriuretic peptides are, preferably, determined in a blood serum or blood plasma sample.

In accordance with the methods of the present invention, determining the amount of a peptide or polypeptide can be achieved by all known means for determining the amount of a peptide in a sample. Said means comprise immunoassay devices and methods which may utilize labeled molecules in various sandwich, competition, or other assay formats. Said assays will develop a signal which is indicative for the presence or absence of the peptide or polypeptide. Moreover, the signal strength can, preferably, be correlated directly or indirectly (e.g. reverse-proportional) to the amount of polypeptide present in a sample. Further suitable methods comprise measuring a physical or chemical property specific for the peptide or polypeptide such as its precise molecular mass or NMR spectrum. Said methods comprise, preferably, biosensors, optical devices coupled to immunoassays, biochips, analytical devices such as mass-spectrometers, NMR- analyzers, or chromatography devices. Further, methods include micro-plate ELISA-based methods, fully-automated or robotic immunoassays (available for example on Elecsys™ analyzers), CBA (an enzymatic Cobalt Binding Assay, available for example on Roche-Hitachi™ analyzers), and latex agglutination assays (available for example on Roche-Hitachi™ analyzers).

Preferably, determining the amount of a peptide or polypeptide comprises the steps of (a) contacting a cell capable of eliciting a cellular response the intensity of which is indicative of the amount of the peptide or polypeptide with the said peptide or polypeptide for an adequate period of time, (b) measuring the cellular response. For measuring cellular responses, the sample or processed sample is, preferably, added to a cell culture and an internal or external cellular response is measured. The cellular response may include the measurable expression of a reporter gene or the secretion of a substance, e.g. a peptide, polypeptide, or a small molecule. The expression or substance shall generate an intensity signal which correlates to the amount of the peptide or polypeptide.

Also preferably, determining the amount of a peptide or polypeptide comprises the step of measuring a specific intensity signal obtainable from the peptide or polypeptide in the sample. As described above, such a signal may be the signal intensity observed at an m/z variable specific for the peptide or polypeptide observed in mass spectra or a NMR spectrum specific for the peptide or polypeptide.

Determining the amount of a peptide or polypeptide may, preferably, comprises the steps of (a) contacting the peptide with a specific ligand, (b) (optionally) removing non-bound ligand, (c) measuring the amount of bound ligand. The bound ligand will generate an intensity signal. Binding includes both covalent and non-covalent binding. A ligand can be any compound, e.g., a peptide, polypeptide, nucleic acid, or small molecule, binding to the peptide or polypeptide described herein. Preferred ligands include antibodies, nucleic acids, peptides or polypeptides such as receptors or binding partners for the peptide or polypeptide and fragments thereof comprising the binding domains for the peptides, and aptamers, e.g. nucleic acid or peptide aptamers. Methods to prepare such ligands are well-known in the art. For example, identification and production of suitable antibodies or aptamers is also offered by commercial suppliers. The person skilled in the art is familiar with methods to develop derivatives of such ligands with higher affinity or specificity. For example, random mutations can be introduced into the nucleic acids, peptides or polypeptides. These derivatives can then be tested for binding according to screening procedures known in the art, e.g. phage display. Antibodies as referred to herein include both polyclonal and monoclonal antibodies, as well as fragments thereof, such as Fv, Fab and F(ab)$_2$ fragments that are capable of binding antigen or hapten. The disclosure includes single chain antibodies and humanized hybrid antibodies wherein amino acid sequences of a non-human donor antibody exhibiting a desired antigen-specificity are combined with sequences of a human acceptor antibody. The donor sequences will usually include at least the antigen-binding amino acid residues of the donor but may comprise other structurally and/or functionally relevant amino acid residues of the donor antibody as well. Such hybrids can be prepared by several methods well known in the art. Preferably, the ligand or agent binds specifically to the peptide or polypeptide. Specific binding means that the ligand or agent should not bind substantially to ("cross-react" with) another peptide, polypeptide or substance present in the sample to be analyzed. Preferably, the specifically bound peptide or polypeptide should be bound with at least 3 times higher, more preferably at least 10 times higher and even more preferably at least 50 times higher affinity than any other relevant peptide or polypeptide. Non-specific binding may be tolerable, if it can still be distinguished and measured unequivocally, e.g. according to its size on a Western Blot, or by its relatively higher abundance in the sample. Binding of the ligand can be measured by any method known in the art. Preferably, said method is semi-quantitative or quantitative. Suitable methods are described in the following.

First, binding of a ligand may be measured directly, e.g. by NMR or surface plasmon resonance.

Second, if the ligand also serves as a substrate of an enzymatic activity of the peptide or polypeptide of interest, an enzymatic reaction product may be measured (e.g. the amount of a protease can be measured by measuring the amount of cleaved substrate, e.g. on a Western Blot). Alternatively, the ligand may exhibit enzymatic properties itself and the "ligand/peptide or polypeptide" complex or the ligand which was bound by the peptide or polypeptide, respectively, maybe contacted with a suitable substrate allowing detection by the generation of an intensity signal. For measurement of enzymatic reaction products, preferably the amount of substrate is saturating. The substrate may also be labeled with a detectable label prior to the reaction. Preferably, the sample is contacted with the substrate for an adequate period of time. An adequate period of time refers to the time necessary for a detectable, preferably measurable, amount of product to be produced. Instead of measuring the amount of product, the time necessary for appearance of a given (e.g. detectable) amount of product can be measured.

Third, the ligand may be coupled covalently or non-covalently to a label allowing detection and measurement of the ligand. Labelling may be done by direct or indirect methods. Direct labelling involves coupling of the label directly (covalently or non-covalently) to the ligand. Indirect labelling involves binding (covalently or non-covalently) of a secondary ligand to the first ligand. The secondary ligand should specifically bind to the first ligand. Said secondary ligand may be coupled with a suitable label and/or be the target (receptor) of tertiary ligand binding to the secondary ligand. The use of secondary, tertiary or even higher order ligands is often used to increase the signal. Suitable secondary and higher order ligands may include antibodies, secondary antibodies, and the well-known streptavidin-biotin system (Vector Laboratories, Inc.). The ligand or substrate may also be "tagged" with one or more tags as known in the art. Such tags may then be targets for higher order ligands. Suitable tags include biotin, digoxygenin, His-Tag, Glutathion-S-Transferase, FLAG, GFP, myc-tag, influenza A virus haemagglutinin (HA), maltose binding protein, and the like. In the case of a peptide or polypeptide, the tag is preferably at the N-terminus and/or C-terminus. Suitable labels are any labels detectable by an appropriate detection method. Typical labels include gold particles, latex beads, acridan ester, luminol, ruthenium, enzymatically active labels, radioactive labels, magnetic labels ("e.g. magnetic beads", including paramagnetic and superparamagnetic labels), and fluorescent labels. Enzymatically active labels include e.g. horseradish peroxidase, alkaline phosphatase, beta-Galactosidase, Luciferase, and derivatives thereof. Suitable substrates for detection include di-amino-benzidine (DAB), 3,3'-5,5'-tetramethylbenzidine, NBT-BCIP (4-nitro blue tetrazolium chloride and 5-bromo-4-chloro-3-indolyl-phosphate, available as ready-made stock solution from Roche Diagnostics), CDP-Star™ (Amersham Biosciences), ECF™ (Amersham Biosciences). A suitable enzyme-substrate combination may result in a colored reaction product, fluorescence or chemoluminescence, which can be measured according to methods known in the art (e.g. using a light-sensitive film or a suitable camera system). As for measuring the enzymatic reaction, the criteria given above apply analogously. Typical fluorescent labels include fluorescent proteins (such as GFP and its derivatives), Cy3 Cy5, Texas Red, Fluorescein, and the Alexa dyes (e.g. Alexa 568). Further fluorescent labels are available e.g. from Molecular Probes (Oregon). Also the use of quantum dots as fluorescent labels is contemplated. Typical radioactive labels include $^{35}$S, $^{125}$I, $^{32}$P, $^{33}$P and the like. A radioactive label can be detected by any method known and appropriate, e.g. a light-sensitive film or a phosphor imager. Suitable measurement methods also include precipitation (particularly immunoprecipitation), electrochemiluminescence (electro-generated chemiluminescence), RIA (radioimmunoassay), ELISA (enzyme-linked immunosorbent assay), sandwich enzyme immune tests, electrochemiluminescence sandwich immunoassays (ECLIA), dissociation-enhanced lanthanide fluoro immuno assay (DELFIA), scintillation proximity assay (SPA), turbidimetry, nephelometry, latex-enhanced turbidimetry or nephelometry, or solid phase immune tests. Further methods known in the art (such as gel electrophoresis, 2D gel electrophoresis, SDS polyacrylamid gel electrophoresis (SDS-PAGE), Western Blotting, and mass spectrometry), can be used alone or in combination with labelling or other detection methods as described above.

The amount of a peptide or polypeptide may be, also preferably, determined as follows: (a) contacting a solid support comprising a ligand for the peptide or polypeptide as specified above with a sample comprising the peptide or polypeptide and (b) measuring the amount peptide or polypeptide which is bound to the support. The ligand, preferably chosen from the group consisting of nucleic acids, peptides, polypeptides, antibodies and aptamers, is preferably present on a solid support in immobilized form. Materials for manufacturing solid supports are well known in the art and include, inter alia, commercially available column materials, polystyrene beads, latex beads, magnetic beads, colloid metal particles, glass and/or silicon chips and surfaces, nitrocellulose strips, membranes, sheets, duracytes, wells and walls of reaction trays, plastic tubes etc. The ligand or agent may be bound to many different carriers. Examples of well-known carriers include glass, polystyrene, polyvinyl chloride, polypropylene, polyethylene, polycarbonate, dextran, nylon, amyloses, natural and modified celluloses, polyacrylamides, agaroses, and magnetite. The nature of the carrier can be either soluble or insoluble. Suitable methods for fixing/immobilizing said ligand are well known and include, but are not limited to ionic, hydrophobic, covalent interactions and the like. It is also contemplated to use "suspension arrays" as arrays (Nolan 2002, Trends Biotechnol. 20(1):9-12). In such suspension arrays, the carrier, e.g. a microbead or microsphere, is present in suspension. The array consists of different microbeads or microspheres, possibly labeled, carrying different ligands. Methods of producing such arrays, for example based on solid-phase chemistry and photo-labile protective groups, are generally known (US 5,744,305).

The term "amount" as used herein encompasses the absolute amount of a polypeptide or peptide, the relative amount or concentration of the said polypeptide or peptide as well as any value or parameter which correlates thereto or can be derived therefrom. Such values or parameters comprise intensity signal values from all specific physical or chemical properties obtained from the said peptides by direct measurements, e.g., intensity values in mass spectra or NMR spectra. Moreover, encompassed are all values or parameters which are obtained by indirect measurements specified elsewhere in this description, e.g., response levels determined from biological read out systems in response to the peptides or intensity signals obtained from specifically bound ligands. It is to be understood that values correlating to the aforementioned amounts or parameters can also be obtained by all standard mathematical operations.

The term "comparing" as used herein encompasses comparing the amount of the peptide or polypeptide comprised by the sample to be analyzed with an amount of a suitable reference source specified elsewhere in this description. It is to be understood that comparing as used herein refers to a comparison of corresponding parameters or values, e.g., an absolute amount is compared to an absolute reference amount while a concentration is compared to a reference concentration or an intensity signal obtained from a test sample is compared to the same type of intensity signal of a reference sample. The comparison referred to in step (b) of the method of the present invention may be carried out manually or computer assisted. For a computer assisted comparison, the value of the determined amount may be compared to values corresponding to suitable references which are stored in a database by a computer program. The computer program may further evaluate the result of the comparison, i.e. automatically provide the desired assessment in a suitable output format. Based on the comparison of the amount determined in step a) and the reference amount, it is possible to assess whether a subject is susceptible for a cardiac therapy and, thus, belongs to the group of subjects which can be successfully treated by the cardiac therapy. Therefore, the reference amount is to be chosen so that either a difference or a similarity in the compared amounts allows identifying those the test subject which belong into the group of subjects susceptible for cardiac therapy or identifying those test subjects which are not susceptible for a cardiac therapy.

The present disclosure further encompasses a device for diagnosing or distinguishing, in a subject having left ventricular hypertrophy, if the subject has physiological left ventricular hypertrophy or suffers from pathological left ventricular hypertrophy; or for distinguishing, in a subject suffering from pathological left ventricular hypertrophy, if the subject suffers from hypertrophic non-obstructive cardiomyopathy, hypertrophic obstructive cardiomyopathy or pressure overload hypertrophy, comprising:

   a) means for determining the amounts of the following peptides:

      a necrosis marker, preferably troponin or a variant thereof;
      a cardiac function marker, preferably a natriuretic peptide or a variant thereof;
      an inflammatory marker, preferably GDF-15 or a variant thereof; and optionally means for determining the amount of P1GF or a variant thereof;

   b) means for comparing the amounts determined in step a) with reference amounts, whereby the pathomechanism(s) of the left ventricular hypertrophy is to be diagnosed.

Depending on the results obtainable by the device, a decision on the adaptation of the therapy may be taken. The therapy may be adapted by e.g. augmenting or diminishing the amounts of the medicaments which are administrated.

The disclosure further encompasses a device for deciding on the therapy in a subject suffering from pathological left ventricular hypertrophy comprising:

   a) means for determining the amounts of the following peptides:

      a necrosis marker, preferably troponin or a variant thereof;
      a cardiac function marker, preferably a natriuretic peptide or a variant thereof;
      an inflammatory marker, preferably GDF-15 or a variant thereof; and optionally means for determining the amount of P1GF or a variant thereof; and

b) means for comparing the amounts determined in step a) with reference amounts, whereby the pathomechanism(s) of the left ventricular hypertrophy is to be diagnosed,

whereby the device is adapted for carrying out the method referred to above.

The present invention further relates to the use of a device, comprising:

- an antibody for determining the amount of troponin T,

- an antibody for determining the amount of a BNP-type marker, in particular of BNP or NT-proBNP;

- an antibody for determining the amount of GDF-15; and

- means for comparing the amounts of the markers with reference amounts;

for diagnosing or distinguishing, in a subject having left ventricular hypertrophy, if the subject has physiological left ventricular hypertrophy or suffers from pathological left ventricular hypertrophy; or
for distinguishing, in a subject suffering from pathological left ventricular hypertrophy, if the subject suffers from hypertrophic non-obstructive cardiomyopathy, hypertrophic obstructive cardiomyopathy or pressure overload hypertrophy; or
for deciding on and/or monitoring the therapy in a subject suffering from pathological left ventricular hypertrophy.

The term "device" as used herein relates to a system of means comprising at least the aforementioned means operatively linked to each other as to allow the prediction. Preferred means for determining the amount of a one of the aforementioned polypeptides as well as means for carrying out the comparison are disclosed above in connection with the method of the invention. How to link the means in an operating manner will depend on the type of means included into the device. For example, where means for automatically determining the amount of the peptides are applied, the data obtained by said automatically operating means can be processed by, e.g., a computer program in order to obtain the desired results. Preferably, the means are comprised by a single device in such a case. Said device may accordingly include an analyzing unit for the measurement of the amount of the peptides or polypeptides in an applied sample and a computer unit for processing the resulting data for the evaluation. The computer unit, preferably, comprises a database including the stored reference amounts or values thereof recited elsewhere in this specification as well as a computer-implemented algorithm for carrying out a comparison of the determined amounts for the polypeptides with the stored reference amounts of the database. Computer-implemented as used herein refers to a computer-readable program code tangibly included into the computer unit. Alternatively, where means such as test stripes are used for determining the amount of the peptides or polypeptides, the means for comparison may comprise control stripes or tables allocating the determined amount to a reference amount. The test stripes are, preferably, coupled to a ligand which specifically binds to the peptides or polypeptides referred to herein. The strip or device, preferably, comprises means for detection of the binding of said peptides or polypeptides to the said ligand. Preferred means for detection are disclosed in connection with embodiments relating to the method of the invention above. In such a case, the means are operatively linked in that the user of the system brings together the result of the determination of the amount and the diagnostic or prognostic value thereof due to the instructions and interpretations given in a manual. The means may appear as separate devices in such an embodiment and are, preferably, packaged together as a kit. The person skilled in the art will realize how to link the means without further ado. Preferred devices are those which can be applied without the particular knowledge of a specialized clinician, e.g., test stripes or electronic devices which merely require loading with a sample. The results may be given as output of raw data which need interpretation by the clinician. Preferably, the output of the device is, however, processed, i.e. evaluated, raw data the interpretation of which does not require a clinician. Further preferred devices comprise the analyzing units/devices (e.g., biosensors, arrays, solid supports coupled to ligands specifically recognizing the natriuretic peptide, Plasmon surface resonance devices, NMR spectrometers, mass-spectrometers etc.) and/or evaluation units/devices referred to above.

Also, the disclosure relates to a device for monitoring the therapy in a subject suffering from pathological left ventricular hypertrophy cardiomyopathy comprising:

a) means for determining the amounts of the following peptides:

a necrosis marker, preferably troponin or a variant thereof;
a cardiac function marker, preferably a natriuretic peptide or a variant thereof;
an inflammatory marker, preferably GDF-15 or a variant thereof; and optionally means for determining the amount of P1GF or a variant thereof; and

b) means for comparing the amounts determined in step a) with reference amounts, whereby the pathomechanism(s) of the left ventricular hypertrophy is to be diagnosed,

whereby the device is adapted for carrying out the method of the present invention referred to above.

The present disclosure also relates to the use of a device or devices as cited beforehand, for:

diagnosing or distinguishing, in a subject having left ventricular hypertrophy, if the subject has physiological left ventricular hypertrophy or suffers from pathological left ventricular hypertrophy; or for

distinguishing, in a subject suffering from pathological left ventricular hypertrophy, if the subject suffers from hypertrophic non-obstructive cardiomyopathy, hypertrophic obstructive cardiomyopathy or pressure overload hypertrophy; or for

deciding on and/or monitoring the therapy in a subject suffering from pathological left ventricular hypertrophy.

The present invention also relates to the use of a kit adapted for carrying out the method of any of claims 1 to 13, comprising:

- an antibody for determining the amount of troponin T,
- an antibody for determining the amount of a BNP-type marker, in of particular BNP or NT-proBNP;
- an antibody for determining the amount of GDF-15,
- means for comparing the amounts determined in step a) with reference amounts of said methods, and
- instructions for carrying out the said method of the present invention;

for diagnosing or distinguishing, in a subject having left ventricular hypertrophy, if the subject has physiological left ventricular hypertrophy or suffers from pathological left ventricular hypertrophy; or

for distinguishing, in a subject suffering from pathological left ventricular hypertrophy, if the subject suffers from hypertrophic non-obstructive cardiomyopathy, hypertrophic obstructive cardiomyopathy or pressure overload hypertrophy; or for deciding on and/or monitoring the therapy in a subject suffering from pathological left ventricular hypertrophy.

Moreover, the disclosure relates to a kit adapted for carrying out the method referred to above comprising:

a) means for determining the amounts of the following peptides:

a necrosis marker, preferably troponin or a variant thereof;
a cardiac function marker, preferably a natriuretic peptide or a variant thereof;
an inflammatory marker, preferably GDF-15 or a variant thereof; and optionally
means for determining the amount of P1GF or a variant thereof; and

b) means for comparing the amounts determined in step a) with reference amounts, whereby the pathomechanism(s) of the left ventricular hypertrophy is to be diagnosed,

whereby the kit is adapted for carrying out the method referred to above. Preferably, the kit comprises instructions for carrying out the said method.

The term "kit" as used herein refers to a collection of the aforementioned means, preferably, provided in separately or within a single container. The container, also preferably, comprises instructions for carrying out the method of the present invention.

The disclosure also relates to the use of a kit or kits as cited beforehand, for:

diagnosing or distinguishing, in a subject having left ventricular hypertrophy, if the subject has physiological left ventricular hypertrophy or suffers from pathological left ventricular hypertrophy; or for

distinguishing, in a subject suffering from pathological left ventricular hypertrophy, if the subject suffers from hypertrophic non-obstructive cardiomyopathy, hypertrophic obstructive cardiomyopathy or pressure overload hypertrophy; or for

deciding on and/or monitoring the therapy in a subject suffering from pathological left ventricular hypertrophy.

The disclosure also relates to the use of: an antibody against at least one marker selected from necrosis markers, at least one marker selected from cardiac function markers and at least one marker selected from inflammatory markers and/or of means for determining the amount of at least one marker selected from necrosis markers, at least one marker selected from cardiac function markers and at least one marker selected from inflammatory markers and/or of means for comparing the amount of at least one marker selected from necrosis markers, at least one marker selected from cardiac function markers and at least one marker selected from inflammatory markers to at least one reference amount, for the manufacture of a diagnostic composition for: diagnosing if the subject has physiological left ventricular hypertrophy or is suffering from pathological left ventricular hypertrophy; distinguishing, in a subject suffering from pathological left ventricular hypertrophy, if the subject suffers from hypertrophic non-obstructive cardiomyopathy, hypertrophic obstructive cardiomyopathy or pressure overload hypertrophy; distinguishing, in a subject suffering from pathological left ventricular hypertrophy, if the subject suffers from hypertrophic non-obstructive cardiomyopathy, on the one hand, or from a cardiomyopathy selected from hypertrophic obstructive cardiomyopathy and pressure overload hypertrophy, on the other hand; deciding on the therapy for treating pathological left ventricular hypertrophy in a subject suffering from the said disease, and/or monitoring a therapy of treating pathological left ventricular hypertrophy in a subject suffering from the said disease.

The disclosure further relates to the use of: at least one marker selected from necrosis markers, at least one marker selected from cardiac function markers and at least one marker selected from inflammatory markers and/or of means for determining the amount of at least one marker selected from necrosis markers, at least one marker selected from cardiac function markers and at least one marker selected from inflammatory markers and/or of means for comparing the amount of at least one marker selected from necrosis markers, at least one marker selected from cardiac function markers and at least one marker selected from inflammatory markers to at least one reference amount for: diagnosing if the subject has physiological left ventricular hypertrophy or is suffering from pathological left ventricular hypertrophy; distinguishing, in a subject suffering from pathological left ventricular hypertrophy, if the subject suffers from hypertrophic non-obstructive cardiomyopathy, hypertrophic obstructive cardiomyopathy or pressure overload hypertrophy; distinguishing, in a subject suffering from pathological left ventricular hypertrophy, if the subject suffers from hypertrophic non-obstructive cardiomyopathy, on the one hand, or from a cardiomyopathy selected from hypertrophic obstructive cardiomyopathy and pressure overload hypertrophy, on the other hand; deciding on the therapy for treating pathological left ventricular hypertrophy in a subject suffering from the said disease, and/or monitoring a therapy of treating pathological left ventricular hypertrophy in a subject suffering from the said disease.

The following Examples shall merely illustrate the invention. They shall not be construed, whatsoever, to limit the scope of the invention.

## Examples

Methods

Troponin T was determined using Roche's electrochemiluminescence ELISA sandwich test Elecsys Troponin T hs (high sensitive) STAT (Short Turn Around Time) assay. The test employs two monoclonal antibodies specifically directed against human cardiac troponin T. The antibodies recognize two epitopes (amino acid position 125-131 and 136-147) located in the central part of the cardiac troponin T protein, which consists of 288 amino acids.

NT-proBNP was determined using Roche's electrochemiluminescence ELISA sandwich test Elecsys proBNP II STAT (Short Turn Around Time) assay. The test employs two monoclonal antibodies which recognize epitopes located in the N-terminal part (1-76) of proBNP (1-108).

To determine the concentration of GDF-15 in serum and plasma samples, an Elecsys prototype test using a polyclonal, GDF-15 affinity chromatography-purified, goat antihuman GDF-15 IgG antibody from R&D Systems (AF957) was developed. In each experiment, a standard curve was generated with recombinant human GDF-15 from R&D Systems (957-GD/CF). The results with new batches or recombinant GDF-15 protein were tested in standard plasma samples and any deviation above 10% was corrected by introducing an adjustment factor for this assay. GDF-15 measurements in serum and plasma samples from the same patient yielded virtually identical results after correction for eventual dilution factors. The detection limit of the assay was 200 pg/ml.

P1GF was determined using Roche's electrochemiluminescence ELISA sandwich test Elecsys P1GF STAT (Short Turn Around Time) assay. The test employs two monoclonal human P1GF specific antibodies (a biotinylated monoclonal antibody and an antibody labeld with a ruthenium complex).

## Example 1:

A collective of 12 healthy competitive athletes having physiological left ventricular hypertrophy were examined during their active training period (i.e. not during holidays or the like where training was timely supended or where the extent of training was reduced relative to the normal training period). In the course of the examination, the serum levels of NT-proBNP, GDF-15 and Troponin T were determined. The following results were found:

**GDF-15:** 397 pg/ml median; (25th percentile: 360 pg/ml; 75th percentile: 442 pg/ml)
**NT-proBNP**:15,72 pg/ml median; (25th percentile: 6,48 pg/ml; 75th percentile: 19,68pg/ml)
**Troponin T**:3,94 pg/ml median; (25th percentile: 3,30 pg/ml; 75th percentile: 6,77 pg/ml)

The values were clearly below those determined in subjects suffering from pathological left ventricular hypertrophy (see example 2) and surprisingly show that healthy individuals suffering from physiological left ventricular hypertrophy can be distinguished from those having pathological hypertrophy on the basis of the above marker detection.

**Example 2:**

In individuals suspected to suffer from heart disease, signs of left ventricular hypertrophy were found by ECG examination. All individuals had a glomerular filtration rate (GFR) exceeding 60 ml/min, patients with renal hypertension were not included in the study.

Thereafter, individuals were diagnosed for hypertensive left ventricular hypertrophy (after exclusion of an aortic stenosis) by echocardiography and determination of blood pressure.

Hypertrophic cardiomyopathy was diagnosed by the exclusion of other known causes which may underlie left ventricular hypertrophy (arterial hypertension, see above; aortic stenosis and athlete's heart; genetic tests to verify the occurrence of hypertrophic cardiomyopathy were not done). In these individuals, obstructive hypertrophic cardiomyopathy was diagnosed if the left ventricular outflow gradient was above 30 mm Hg.

In all individuals, blood samples were taken and the amounts/levels of the respective peptides were determined, in serum samples as described in Example 1.

Patients diagnosed with obstructive hypertrophic cardiomyopathy suspected to suffer from a progressive disease and who were refractory in respect to medicament therapy, were treated by ablation after previous probatory occlusion of the feeding vessels. Blood samples were taken prior to and 6 months after intervention.

Table 1

| | NT-proBNP (pg/ml) | | | hsTnT (pg/ml) | | |
|---|---|---|---|---|---|---|
| | N=19 HyN CMP | N=11 Hob CMP | N=12 Hyt CMP | N=19 HyN CMP | N=11 Hob CMP | N=12 Hyt CMP |
| Median | 351.0 | 229.0 | 203.6 | 8.2 | 7.8 | 11.8 |
| 75th perc. | 1084.4 | 1283.5 | 624.3 | 30.4 | 17.7 | 22.3 |
| 25th perc. | 248.0 | 111.4 | 73.0 | 5.8 | 2.4 | 6.8 |

| | P1GF (pg/ml) | | | GDF-15 (pg/ml) | | |
|---|---|---|---|---|---|---|
| | N=19 HyN CMP | N=11 Hob CMP | N=12 Hyt CMP | N=19 HyN CMP | N=11 Hob CMP | N=12 Hyt CMP |
| Median | 8.9 | 15.0 | 10.8 | 969.2 | 1030.6 | 1387.2 |
| 75th perc. | 13.1 | 16.7 | 13.3 | 1657.3 | 2046.3 | 1628.0 |
| 25th perc. | 7.0 | 12.4 | 9.4 | 721.2 | 955.2 | 1110.7 |

Table 2

| | N=19 Hypertrophic CMP | N=11 Hypertrophic Obstructive CMP | N=12 Hyt CMP | N=19 Hypertrophic CMP | N=11 Hypertrophic Obstructive CMP | N=12 Hyt CMP |
|---|---|---|---|---|---|---|
| | proBNP / GDF-15 Ratio | proBNP / GDF-15 Ratio | proBNP / GDF-15 Ratio | hsTnT / GDF-15 Ratio | hsTnT / GDF-15 Ratio | hsTnT / GDF-15 Ratio |
| Median | 0.450 | 0.200 | 0.190 | 0.011194 | 0.002559 | 0.007880 |
| 75th perc. | 0.820 | 0.430 | 0.420 | 0.005628 | 0.002200 | 0.004787 |

(continued)

| | N=19 Hypertrophic CMP | N=11 Hypertrophic Obstructive CMP | N=12 Hyt CMP | N=19 Hypertrophic CMP | N=11 Hypertrophic Obstructive CMP | N=12 Hyt CMP |
|---|---|---|---|---|---|---|
| | proBNP / GDF-15 Ratio | proBNP / GDF-15 Ratio | proBNP / GDF-15 Ratio | hsTnT / GDF-15 Ratio | hsTnT / GDF-15 Ratio | hsTnT / GDF-15 Ratio |
| 25th perc. | 0.190 | 0.160 | 0.080 | 0.026497 | 0.006135 | 0.013570 |

Table 3

| TASH N=71 Pat. | NT-proBNP (pg/ml) | | hs TnT (pg/ml) | | GDF-15 (pg/ml) | | P1GF (pg/ml) | |
|---|---|---|---|---|---|---|---|---|
| | prae-TASH | After 6 month | prae-TASH | After 6 month | prae-TASH | After 6 month | prae-TASH | After 6 month |
| Median | 777.78 | 334.35 | 10.42 | 10.00 | 1079.68 | 1099.93 | 15.99 | 16.38 |
| 75th perc. | 1272.94 | 799.26 | 17.26 | 19.06 | 1372.7 | 1500.42 | 18.89 | 19.13 |
| 25th perc. | 190.67 | 126.71 | 6.09 | 5.69 | 817.28 | 792.86 | 13.39 | 14.17 |

In the tables 1, 2 and 3, the following abbreviations are used:

**HyN CMP: hypertrophic cardiomyopathy (non-obstructive);**
**Hob CMP: hypertrophic obstructive cardiomyopathy;**
**Hyt CMP: hypertensive left ventricular hypertrophy**

N is the number of patients enrolled in the study (19 for HyN CMP; 11 for Hob CMP; 12 for Hyt CMP. The amounts of P1GF and GDF-15, NT-proBNP and Troponin T were determined in serum samples.
The tables show:

**Table 1:** Concentrations of NT-proBNP, high sensitive Troponin T, P1GF and GDF-15 in patients with hypertrophic cardiomyopathy, hypertrophic obstructive cardiomyopathy and hypertensive left ventricular hypertrophy.
The 75th and 25th percentiles and the medians are indicated.

**Table 2:** ratios of NT-proBNP/GDF-15 and of high sensitive Troponin T/GDF-15 in patients with hypertrophic cardiomyopathy, hypertrophic obstructive cardiomyopathy and hypertensive left ventricular hypertrophy.
The 75th and 25th percentiles and the medians are indicated.

**Table 3:** Concentrations of NT-proBNP, high sensitive Troponin T, P1GF and GDF-15 in patients with hypertrophic obstructive cardiomyopathy, before TASH treatment (prae-TASH) and 6 months after TASH treatment.
The 75th and 25th percentiles and the medians are indicated.

The results are depicted in tables 1 and 2 show that the various forms of left ventricular hypertrophy can be distinguished via the determination of the levels/amounts of the peptides cited in the tables.
Furthermore, by a comparison of the values depicted in tables 1 and 2 with the values obtained from healthy individuals (see example 1), it is shown that it is possible to distinguish between these healthy individuals and individuals suffering from pathological left ventricular hypertrophy.
The values depicted in table 1 show that, relative to prae-TASH, the level of NT-proBNP was lower after a successful TASH therapy and that NT-proBNP can be used as indicator for a successful TASH treatment.

**Example 3:**

In individuals suspected to suffer from heart disease, signs of hypertrophy were found by ECG examination.

All individuals had a GFR exceeding 60 ml/min, patients with renal hypertension were not included in the study.

Thereafter, individuals were diagnosed for the various form of hypertrophy as described in Example 2, and the amounts of P1GF were determined. The following results were found:

**Hob CMP:** 15.0 pg/ml median; (25th percentile: 12.4 pg/ml; 75th percentile: 16.7 pg/ml)
**HyN CMP:** 8.9 pg/ml median; (25th percentile: 7.0 pg/ml; 75th percentile: 13.1 pg/ml)
**Hyt CMP:** 10.8 pg/ml median; (25th percentile: 9.4 pg/ml; 75th percentile: 13.3 pg/ml)

The results show that subjects suffering from hypertrophic obstructive cardiomyopathy show the highest amounts/levels of P1GF. This gives additional information on the form of hypertrophy the individual suffers from and allows to rule in hypertrophic cardiomyopathy, in addition to the information provided by the amounts of the 3 other marker NT-proBNP, high sensitive Troponin T, and GDF-15 and, respectively, the ratios thereof. The amount/level of P1GF even allows to diagnose hypertrophic obstructive cardiomyopathy in an individual, without referring to the other markers.

## Claims

1. A method for diagnosing or distinguishing, in a subject having left ventricular hypertrophy, if the subject has physiological left ventricular hypertrophy or suffers from pathological left ventricular hypertrophy, the method comprising the steps of

    a) determining the amounts of troponin T, of at least one BNP-type marker, in particular BNP or NT-proBNP, and of GDF-15 (Growth Differentiation Factor 15), in at least one sample of said subject,
    b) comparing the amounts of the said markers as determined in step a) to suitable reference amounts, and
    c) diagnosing if the subject has physiological left ventricular hypertrophy or is suffering from pathological left ventricular hypertrophy.

2. The method according to claim 1, wherein the following values are indicative for a subject having pathological left ventricular hypertrophy: troponin T: > about 5 pg/ml; BNP-type marker, preferably BNP or NT-proBNP, in particular NT-proBNP: > about 75 pg/ml; GDF-15: > about 600 pg/ml.

3. A method for distinguishing, in a subject suffering from pathological left ventricular hypertrophy, if the subject suffers from hypertrophic non-obstructive cardiomyopathy, hypertrophic obstructive cardiomyopathy or pressure overload hypertrophy, comprising the steps of

    a) determining the amounts of troponin T, of at least one BNP-type marker, in particular BNP or NT-proBNP, and of GDF-15, in at least one sample of said subject,
    b) comparing the amounts as determined in step a) to reference amounts, and
    c) distinguishing between hypertrophic non-obstructive cardiomyopathy, hypertrophic obstructive cardiomyopathy and pressure overload hypertrophy, depending on the results of step b).

4. A method for distinguishing, in a subject suffering from pathological left ventricular hypertrophy, if the subject suffers from hypertrophic non-obstructive cardiomyopathy, on the one hand, or from a cardiomyopathy selected from hypertrophic obstructive cardiomyopathy and pressure overload hypertrophy, on the other hand, comprising the steps of

    a) determining the amounts of at least one BNP-type marker, in particular BNP or NT-proBNP, and of GDF-15, in at least one sample of said subject,
    b) forming a ratio between a BNP-type marker and GDF-15 as determined in step a),
    c) comparing the ratio as determined in step b) to reference ratios, and
    d) distinguishing between hypertrophic non-obstructive cardiomyopathy, on the one hand, and a cardiomyopathy selected from hypertrophic obstructive cardiomyopathy, and pressure overload hypertrophy, on the other hand, depending on the results of step c).

5. The method according to claim 4, wherein NT-proBNP/GDF-15 ratio values indicative for the occurrence of hypertrophic non-obstructive cardiomyopathy are values $\geq$ about 0.43, preferably $\geq$ 0.43, and/or wherein NT-proBNP/GDF-15 ratio values indicative for the occurrence of hypertrophic obstructive cardiomyopathy and pressure overload hypertrophy are values up to about 0.43, preferably < 0.43.

6. A method for distinguishing, in a subject suffering from pathological left ventricular hypertrophy, if the subject suffers from hypertrophic non-obstructive cardiomyopathy, hypertrophic obstructive cardiomyopathy or pressure overload hypertrophy, comprising the steps of

   a) determining the amounts of troponin T, and of GDF-15, in at least one sample of said subject,
   b) forming a ratio between troponin T and GDF-15 as determined in step a),
   c) comparing the ratio as determined in step b) to reference ratios, and
   d) distinguishing between hypertrophic non-obstructive cardiomyopathy, hypertrophic obstructive cardiomyopathy and pressure overload hypertrophy, depending on the results of step c).

7. The method according to claim 6, wherein troponin T/GDF-15 ratio values indicative for the occurrence of hypertrophic non-obstructive cardiomyopathy are values ≥ about 0.01, preferably values > 0.01, wherein troponin T/GDF-15 ratio values indicative for the occurrence of hypertrophic obstructive cardiomyopathy and pressure overload hypertrophy are values ≤ about 0.004, preferably values ≤ 0.004, and/or wherein troponin T/GDF-15 ratio values indicative for the occurrence of pressure overload hypertrophy are values ranging from > about 0.004 to < about 0.01.

8. A method of deciding on the therapy for treating pathological left ventricular hypertrophy in a subject suffering from the said disease, comprising the steps of

   a) determining the amounts of troponin T, of at least one BNP-type marker, in particular BNP or NT-proBNP, and of GDF-15, in at least one sample of said subject,
   b) comparing the amounts of the said markers as determined in step a) to suitable reference amounts, and
   c) deciding on the therapy, based on the comparison carried out in step b).

9. The method according to claim 8, wherein the BNP-type marker is NT-proBNP and an amount of NT-proBNP of > about 300 pg/ml indicates that agents effecting cardiac function, preferably selected from: beta blockers; nitrates; adrenergic agonists; positive inotropic agents; diuretics; should be administered, an amount of GDF-15 of ≥ about 800 pg/ml indicates that anti-inflammatory drugs; angiotensin receptor antagonists and aldosterone antagonists; and/or statines; should be administered, and/or wherein an amount of Troponin T of ≥ about 3 pg/ml indicates that percutane coronary intervention should be carried out.

10. A method of monitoring a therapy of treating pathological left ventricular hypertrophy in a subject suffering from the said disease, comprising the steps of

   a) determining the amounts of troponin T, of at least one BNP-type marker, in particular BNP or NT-proBNP, and of GDF-15, in at least one sample of said subject,
   b) comparing the amounts of the said markers as determined in step a) to suitable reference amounts, and
   c) adapting or discontinuing the therapy, based on the comparison carried out in step b).

11. The method according to claim 10, wherein the reference amounts are the amounts cited in claim 9, or amounts determined before the therapy was initiated.

12. The method according to claim 10 or 11, wherein a decrease or increase of 20% of the determined amounts in respect to the reference amounts, in case of a decrease of the marker amounts is indicative for an amelioration or, in case of an increase of the marker amounts is indicative of a deterioration of the pathological state of the subject, and/or wherein transcoronary ablation of septum hypertrophy (TASH) has been carried out in a subject suffering from obstructive hypertrophic cardiomyopathy, a natriuretic peptide, in particular NT-proBNP, is determined and a decrease of 20% in respect to the reference value is indicative of a successful TASH intervention.

13. The method according to any one of claims 3 to 12, wherein additionally the amount of P1GF is determined.

14. Use of a device, comprising:

   - an antibody for determining the amount of troponin T,
   - an antibody for determining the amount of a BNP-type marker, in particular of BNP or NT-proBNP;
   - an antibody for determining the amount of GDF-15; and
   - means for comparing the amounts of the markers with reference amounts;

for diagnosing or distinguishing, in a subject having left ventricular hypertrophy, if the subject has physiological left ventricular hypertrophy or suffers from pathological left ventricular hypertrophy; or

for distinguishing, in a subject suffering from pathological left ventricular hypertrophy, if the subject suffers from hypertrophic non-obstructive cardiomyopathy, hypertrophic obstructive cardiomyopathy or pressure overload hypertrophy; or

for deciding on and/or monitoring the therapy in a subject suffering from pathological left ventricular hypertrophy.

15. Use of a kit adapted for carrying out the method of any of claims 1 to 13, comprising:

    - an antibody for determining the amount of troponin T,
    - an antibody for determining the amount of a BNP-type marker, in of particular BNP or NT-proBNP;
    - an antibody for determining the amount of GDF-15,
    - means for comparing the amounts determined in step a) with reference amounts of said methods, and
    - instructions for carrying out the said method of the present invention;

for diagnosing or distinguishing, in a subject having left ventricular hypertrophy, if the subject has physiological left ventricular hypertrophy or suffers from pathological left ventricular hypertrophy; or

for distinguishing, in a subject suffering from pathological left ventricular hypertrophy, if the subject suffers from hypertrophic non-obstructive cardiomyopathy, hypertrophic obstructive cardiomyopathy or pressure overload hypertrophy; or

for deciding on and/or monitoring the therapy in a subject suffering from pathological left ventricular hypertrophy.

## Patentansprüche

1. Verfahren zur Diagnose oder Unterscheidung bei einem Patienten mit linksventrikulärer Hypertrophie, ob der Patient eine physiologische linksventrikuläre Hypertrophie hat oder an einer pathologischen linksventrikulären Hypertrophie leidet, wobei das Verfahren die folgenden Schritte umfasst:

    a) die Bestimmung der Mengen von Troponin T, von mindestens einem Marker vom BNP-Typ, insbesondere BNP oder NT-proBNP und von GDF-15 (Growth Differentiation Factor 15) in mindestens einer Probe des Patienten,
    b) den Vergleich der Mengen der in Schritt a) bestimmten Marker mit geeigneten Vergleichsmengen und
    c) die Diagnose, ob der Patient eine physiologische linksventrikuläre Hypertrophie hat oder an einer pathologischen linksventrikulären Hypertrophie leidet.

2. Verfahren nach Anspruch 1, wobei die folgenden Werte indikativ für einen Patienten mit pathologischer linksventrikulärer Hypertrophie sind: Troponin T: > etwa 5 pg/ml, Marker vom BNP-Typ, vorzugsweise BNP oder NT-proBNP, insbesondere NT-proBNP: > etwa 75 pg/ml, GDF-15: > etwa 600 pg/ml.

3. Verfahren zum Unterscheiden, in einem an einer pathologischen linksventrikulären Hypertrophie leidenden Patienten, ob der Patient an hypertropher nichtobstruktiver Kardiomyopathie, hypertropher obstruktiver Kardiomyopathie oder Drucküberlastungshypertrophie leidet, welches die folgenden Schritte umfasst:

    a) die Bestimmung der Mengen von Troponin T, von mindestens einem Marker vom BNP-Typ, insbesondere BNP oder NT-proBNP, und von GDF-15 in mindestens einer Probe des Patienten,
    b) den Vergleich der wie in Schritt a) bestimmten Mengen mit Vergleichsmengen und
    c) die Unterscheidung zwischen hypertropher nichtobstruktiver Kardiomyopathie, hypertropher obstruktiver Kardiomyopathie und Drucküberlastungshypertrophie, in Abhängigkeit von den Ergebnissen von Schritt b).

4. Verfahren zur Unterscheidung, bei einem an pathologischer linksventrikulärer Hypertrophie leidenden Patienten, ob der Patient an hypertropher nichtobstruktiver Kardiomyopathie einerseits oder an einer Kardiomyopathie ausgewählt aus hypertropher obstruktiver Kardiomyopathie und Drucküberlastungshypertrophie andererseits leidet, welches die folgenden Schritte umfasst:

    a) die Bestimmung der Mengen von mindestens einem Marker vom BNP-Typ, insbesondere BNP oder NT-proBNP, und von GDF-15, in mindestens einer Probe des Patienten,
    b) das Bilden eines Verhältnisses zwischen einem Marker vom BNP-Typ und GDF-15, wie in Schritt a) bestimmt,

c) den Vergleich des wie in Schritt b) bestimmten Verhältnisses mit Vergleichsverhältnissen und
d) die Unterscheidung zwischen hypertropher nichtobstruktiver Kardiomyopathie einerseits und einer Kardiomyopathie ausgewählt aus hypertropher obstruktiver Kardiomyopathie und Drucküberlastungshypertrophie andererseits, in Abhängigkeit von den Ergebnissen von Schritt c).

5. Verfahren nach Anspruch 4, wobei NT-proBNP/GDF-15-Verhältniswerte $\geq$ etwa 0,43, vorzugsweise $\geq$ 0,43, indikativ für das Vorhandensein einer hypertrophen nichtobstruktiven Kardiomyopathie sind und/oder wobei NT-proBNP/GDF-15-Verhältniswerte von bis zu etwa 0,43, vorzugsweise < 0,43, indikativ für das Vorhandensein von hypertropher obstruktiver Kardiomyopathie und Drucküberlastungshypertrophie sind.

6. Verfahren zum Unterscheiden, in einem an pathologischer linksventrikulärer Hypertrophie leidenden Patienten, ob der Patient an hypertropher nichtobstruktiver Kardiomyopathie, hypertropher obstruktiver Kardiomyopathie oder Drucküberlastungshypertrophie leidet, welches die folgenden Schritte umfasst:

a) die Bestimmung der Mengen an Troponin T und an GDF-15 in mindestens einer Probe des Patienten,
b) das Bilden eines Verhältnisses zwischen Troponin T und GDF-15, wie in Schritt a) bestimmt,
c) das Vergleichen des wie in Schritt b) bestimmten Verhältnisses mit Vergleichsverhältnissen und
d) die Unterscheidung zwischen hypertropher nichtobstruktiver Kardiomyopathie, hypertropher obstruktiver Kardiomyopathie und Drucküberlastungshypertrophie, in Abhängigkeit von den Ergebnissen von Schritt c).

7. Verfahren nach Anspruch 6, wobei Troponin T/GDF-15-Verhältniswerte von $\geq$ etwa 0,01, vorzugsweise Werte $\geq$ 0,01, indikativ für das Vorhandensein von hypertropher nichtobstruktiver Kardiomyopathie sind, wobei Troponin T/GDF-15-Verhältniswerte von $\leq$ etwa 0,004, vorzugsweise Werte $\leq$ 0,004, indikativ für das Vorhandensein von hypertropher obstruktiver Kardiomyopathie und Drucküberlastungshypertrophie sind und/oder wobei Troponin T/GDF-15-Verhältniswerte von > etwa 0,004 bis < etwa 0,01 indikativ für das Vorhandensein von Drucküberlastungshypertrophie sind.

8. Verfahren zum Entscheiden hinsichtlich einer Therapie zur Behandlung pathologischer linksventrikulärer Hypertrophie in einem an der Krankheit leidenden Patienten, welches die folgenden Schritte umfasst:

a) die Bestimmung der Mengen von Troponin T, von mindestens einem Marker vom BNP-Typ, insbesondere BNP oder NT-proBNP, und von GDF-15 in mindestens einer Probe des Patienten,
b) das Vergleichen der Mengen der wie im Schritt a) bestimmten Marker mit geeigneten Referenzmengen und
c) die Entscheidung bezüglich der Therapie, basierend auf dem in Schritt b) durchgeführten Vergleich.

9. Verfahren nach Anspruch 8, wobei es sich bei dem Marker vom BNP-Typ um NT-proBNP handelt und eine Menge an NT-proBNP von $\geq$ etwa 300 pg/ml darauf hindeutet, dass die Herzfunktion beeinflussende Mittel, vorzugsweise ausgewählt aus: Betablockern, Nitraten, adrenärgen Agonisten, positivionotropischen Mitteln und Diuretika verabreicht werden sollten, eine Menge an GDF-15 von $\geq$ etwa 800 pg/ml darauf hindeutet, dass entzündungshemmende Arzneimittel, Angiotensinrezeptorantagonisten und Aldosteronantagonisten und/oder Statine verabreicht werden sollten und/oder wobei eine Menge an Troponin T von $\geq$ etwa 3 pg/ml darauf hindeutet, dass ein perkutaner koronarer Eingriff durchgeführt werden sollte.

10. Verfahren zur Kontrolle einer Therapie zur Behandlung pathologischer linksventrikulärer Hypertrophie bei einem an der Krankheit leidenden Patienten, welches die folgenden Schritte umfasst:

a) die Bestimmung der Mengen von Troponin T, von mindestens einem Marker vom BNP-Typ, insbesondere BNP oder NT-proBNP und von GDF-15 in mindestens einer Probe des Patienten,
b) den Vergleich der wie in Schritt a) bestimmten Mengen der Marker mit geeigneten Vergleichsmengen und
c) das Anpassen oder Absetzen der Therapie, basierend auf dem in Schritt b) durchgeführten Vergleich.

11. Verfahren nach Anspruch 10, wobei es sich bei den Vergleichsmengen um die in Anspruch 9 angeführten Mengen oder vor Beginn der Therapie bestimmte Mengen handelt.

12. Verfahren nach Anspruch 10 oder 11, wobei eine Verminderung oder Zunahme von 20% der bestimmten Mengen, bezogen auf die Vergleichsmengen, bei einer Abnahme der Mengen an Marker indikativ für eine Verbesserung oder bei einer Zunahme der Mengen an Marker indikativ für eine Verschlechterung des pathologischen Zustands des Patienten ist und/oder wobei eine transkoronare Ablation einer Septumhypertrophie (Transcoronary Ablation of Septum Hypertrophy, TASH) an einem an obstruktiver hypertropher Kardiomyopathie leidenden Patienten durchgeführt wurde, ein natriuretisches Peptid, insbesondere NT-proBNP, bestimmt wird und eine Verminderung von 20%, bezogen auf den Vergleichswert, indikativ für einen erfolgreichen TASH-Eingriff ist.

**13.** Verfahren nach einem der Ansprüche 3 bis 12, wobei zusätzlich die Menge an P1GF bestimmt wird.

**14.** Verwendung eines Geräts, umfassend:

- einen Antikörper zur Bestimmung der Menge von Troponin T,
- einen Antikörper zur Bestimmung der Menge eines Markers vom BNP-Typ, insbesondere BNP oder NT-proBNP,
- einen Antikörper zur Bestimmung der Menge von GDF-15 und
- Mittel zum Vergleich der Mengen der Marker mit Vergleichsmengen,

zur Diagnose oder Unterscheidung, bei einem Patienten mit linksventrikulärer Hypertrophie, ob der Patient physiologische linksventrikuläre Hypertrophie hat oder an pathologischer linksventrikulärer Hypertrophie leidet, oder zum Unterscheiden, bei einem an pathologischer linksventrikulärer Hypertrophie leidenden Patienten, ob der Patient an hypertropher nichtobstruktiver Kardiomyopathie, hypertropher obstruktiver Kardiomyopathie oder Drucküberlastungshypertrophie leidet, oder zum Entscheiden bezüglich der Therapie und/oder zur Kontrolle der Therapie in einem an pathologischer linksventrikulärer Hypertrophie leidenden Patienten.

**15.** Verwendung eines für das Durchführen des Verfahrens nach einem der Ansprüche 1 bis 13 ausgelegten Kits, umfassend:

- einen Antikörper zur Bestimmung der Menge an Troponin T,
- einen Antikörper zur Bestimmung der Menge eines Markers vom BNP-Typ, insbesondere BNP oder NT-proBNP,
- einen Antikörper zur Bestimmung der Menge an GDF-15,
- Mittel zum Vergleichen der in Schritt a) bestimmten Mengen mit Vergleichsmengen der Verfahren und
- Anleitungen zum Ausführen des Verfahrens der vorliegenden Erfindung,

zur Diagnose oder Unterscheidung, bei einem Patienten mit linksventrikulärer Hypertrophie, ob der Patient physiologische linksventrikuläre Hypertrophie hat oder an pathologischer linksventrikulärer Hypertrophie leidet, oder zum Unterscheiden, bei einem an pathologischer linksventrikulärer Hypertrophie leidenden Patienten, ob der Patient an hypertropher nichtobstruktiver Kardiomyopathie, hypertropher obstruktiver Kardiomyopathie oder Drucküberlastungshypertrophie leidet, oder zum Entscheiden bezüglich der Therapie und/oder zur Kontrolle der Therapie in einem an pathologischer linksventrikulärer Hypertrophie leidenden Patienten.

**Revendications**

**1.** Procédé pour diagnostiquer ou discerner, chez un sujet victime d'une hypertrophie ventriculaire gauche, le fait de savoir si le sujet est victime d'une hypertrophie ventriculaire gauche de type physiologique ou souffre d'une hypertrophie ventriculaire gauche de type pathologique, le procédé comprenant les étapes dans lesquelles :

a) on détermine les quantités de troponine T, d'au moins un marqueur de type BNP, en particulier du BNP ou du NT-proBNP, et du GDF-15 (facteur de croissance et de différenciation 15), dans au moins un échantillon dudit sujet ;
b) on compare les quantités desdits marqueurs comme déterminé à l'étape a) à des quantités de référence appropriées ; et
c) on pose un diagnostic quant au fait de savoir si le sujet est victime d'une hypertrophie ventriculaire gauche de type physiologique ou souffre d'une hypertrophie ventriculaire gauche de type pathologique.

**2.** Procédé selon la revendication 1, dans lequel les valeurs suivantes sont le signe qu'un sujet est victime d'une hypertrophie ventriculaire gauche de type pathologique : troponine T : > environ 5 pg/ml ; marqueur de type BNP, de préférence BNP ou NT-proBNP, en particulier NT-proBNP : > environ 75 pg/ml ; GDF-15 : > environ 600 pg/ml.

3. Procédé pour discerner, chez un sujet souffrant d'une hypertrophie ventriculaire gauche de type pathologique, le fait de savoir si le sujet souffre d'une cardiomyopathie hypertrophique non obstructive, d'une cardiomyopathie hypertrophique obstructive ou d'une hypertrophie due à une surcharge de pression, comprenant les étapes dans lesquelles :

   a) on détermine les quantités de troponine T, d'au moins un marqueur de type BNP, en particulier du BNP ou du NT-proBNP, et du GDF-15, dans au moins un échantillon dudit sujet ;
   b) on compare les quantités desdits marqueurs comme déterminé à l'étape a) à des quantités de référence ; et
   c) on opère une distinction entre une cardiomyopathie hypertrophique non obstructive, une cardiomyopathie hypertrophique obstructive et une hypertrophie due à une surcharge de pression, en fonction des résultats que l'on obtient à l'étape b).

4. Procédé pour discerner, chez un sujet souffrant d'une hypertrophie ventriculaire gauche de type pathologique, le fait de savoir si le sujet souffre d'une cardiomyopathie hypertrophique non obstructive, d'une part, ou d'une cardiomyopathie choisie parmi une cardiomyopathie hypertrophique obstructive et une hypertrophie due à une surcharge de pression, d'autre part, comprenant les étapes dans lesquelles :

   a) on détermine les quantités d'au moins un marqueur de type BNP, en particulier du BNP ou du NT-proBNP, et du GDF-15, dans au moins un échantillon dudit sujet ;
   b) on établit un rapport entre un marqueur de type BNP et le GDF-15 comme déterminé à l'étape a) ;
   c) on compare le rapport tel que déterminé à l'étape b) à des rapports de référence ; et
   d) on opère une distinction entre une cardiomyopathie hypertrophique non obstructive, d'une part, et une cardiomyopathie choisie parmi une cardiomyopathie hypertrophique obstructive et une hypertrophie due à une surcharge de pression, d'autre part, en fonction des résultats que l'on obtient à l'étape c).

5. Procédé selon la revendication 4, dans lequel les valeurs du rapport NT-proBNP/GDF-15 qui sont le signe de l'occurrence d'une cardiomyopathie hypertrophique non obstructive sont des valeurs ≥ environ 0,43, de préférence ≥ 0,43, et/ou dans lequel les valeurs du rapport NT-proBNP/GDF-15 qui sont le signe de l'occurrence d'une cardiomyopathie hypertrophique obstructive et d'une hypertrophie due à une surcharge de pression sont des valeurs s'élevant jusqu'à environ 0,43, de préférence < 0,43.

6. Procédé pour discerner, chez un sujet souffrant d'une hypertrophie ventriculaire gauche de type pathologique, le fait de savoir si le sujet souffre d'une cardiomyopathie hypertrophique non obstructive, cardiomyopathie hypertrophique obstructive ou d'une hypertrophie due à une surcharge de pression, comprenant les étapes dans lesquelles :

   a) on détermine les quantités de troponine T et du GDF-15, dans au moins un échantillon dudit sujet ;
   b) on établit un rapport entre la troponine T et le GDF-15 comme déterminé à l'étape a) ;
   c) on compare le rapport tel que déterminé à l'étape b) à des rapports de référence ; et
   d) on opère une distinction entre une cardiomyopathie hypertrophique non obstructive, une cardiomyopathie hypertrophique obstructive et une hypertrophie due à une surcharge de pression, en fonction des résultats que l'on obtient à l'étape c).

7. Procédé selon la revendication 6, dans lequel des valeurs du rapport troponine T/GDF-15 qui sont le signe de l'occurrence d'une cardiomyopathie hypertrophique non obstructive sont des valeurs ≥ environ 0,01, de préférence des valeurs ≥ 0,01, dans lequel des valeurs du rapport troponine T/GDF-15 qui sont le signe de l'occurrence d'une cardiomyopathie hypertrophique obstructive et d'une hypertrophie due à une surcharge de pression sont des valeurs ≤ environ 0,004, de préférence des valeurs ≤ 0,004, et/ou dans lequel des valeurs du rapport troponine T/GDF-15 qui sont le signe de l'occurrence d'une hypertrophie due à une surcharge de pression sont des valeurs qui se situent dans la plage de > environ 0,004 à < environ 0,01.

8. Procédé pour prendre une décision quant à la thérapie destinée à traiter une hypertrophie ventriculaire gauche de type pathologique chez un sujet souffrant de ladite maladie, comprenant les étapes dans lesquelles :

   a) on détermine les quantités de troponine T, d'au moins un marqueur de type BNP, en particulier du BNP ou du NT-proBNP, et du GDF-15, dans au moins un échantillon dudit sujet ;
   b) on compare les quantités desdits marqueurs comme déterminé à l'étape a) à des quantités de référence appropriées ; et
   c) on prend une décision concernant la thérapie, en se basant sur la comparaison mise en oeuvre l'étape b).

**9.** Procédé selon la revendication 8, dans lequel le marqueur de type BNP est le NT-proBNP et dans lequel une quantité de NT-proBNP ≥ environ 300 pg/ml est le signe que des agents affectant les fonctions cardiaques choisies de préférence parmi : des bêtabloquants ; des nitrates ; des agonistes adrénergiques ; des agents inotropes positifs ; des diurétiques, doivent être administrés ; dans lequel une quantité de GDF-15 ≥ environ 800 pg/ml est le signe que des agents anti-inflammatoires ; des antagonistes du récepteur de l'angiotensine et des antagonistes de l'aldostérone ; et/ou des statines doivent être administrés ; et/ou dans lequel une quantité de troponine T ≥ environ 3 pg/ml est le signe qu'une intervention coronaire percutanée doit être mise en oeuvre.

**10.** Procédé de surveillance d'une thérapie pour le traitement d'une hypertrophie ventriculaire gauche de type pathologique chez un sujet souffrant de ladite maladie, comprenant les étapes dans lesquelles :

a) on détermine les quantités de troponine T, d'au moins un marqueur de type BNP, en particulier du BNP ou du NT-proBNP, et du GDF-15, dans au moins un échantillon dudit sujet ;
b) on compare les quantités desdits marqueurs comme déterminé à l'étape a) à des quantités de référence appropriées ; et
c) on adapte ou on interrompt la thérapie en se basant sur la comparaison mise en oeuvre à l'étape b).

**11.** Procédé selon la revendication 10, dans lequel les quantités de référence représentent les quantités indiquées à la revendication 9 ou des quantités déterminées avant le déclenchement de la thérapie.

**12.** Procédé selon la revendication 10 ou 11, dans lequel une diminution ou une augmentation de 20 % des quantités déterminées par rapport au quantités de référence, dans le cas d'une diminution des quantités des marqueurs, est un signe d'une amélioration ou, dans le cas d'une augmentation des quantités des marqueurs, est le signe d'une détérioration de l'état pathologique du sujet, et/ou lorsqu'une ablation transcoronaire de l'hypertrophie septale (TASH) a été mis en oeuvre chez un sujet souffrant d'une cardiomyopathie hypertrophique obstructive, on détermine un peptide natriurétique en particulier le NT-proBNP, et une diminution de 20 % par rapport à la valeur de référence est le signe d'une intervention TASH couronnée de succès.

**13.** Procédé selon l'une quelconque des revendications 3 à 12, dans lequel on détermine en outre la quantité du PIGF.

**14.** Utilisation d'un dispositif, comprenant :

- un anticorps pour déterminer la quantité de troponine T ;
- un anticorps pour déterminer la quantité d'un marqueur de type BNP, en particulier du BNP ou du NT-proBNP ;
- un anticorps pour déterminer la quantité du GDF-15 ; et
- des moyens pour comparer les quantités des marqueurs à des quantités de référence ;

pour diagnostiquer ou discerner, chez un sujet victime d'une hypertrophie ventriculaire gauche, le fait de savoir si le sujet est victime d'une hypertrophie ventriculaire gauche de type physiologique ou souffre d'une hypertrophie ventriculaire gauche de type pathologique ;
pour discerner, chez un sujet souffrant d'une hypertrophie ventriculaire gauche de type pathologique, le fait de savoir si le sujet souffre d'une cardiomyopathie hypertrophique non obstructive, d'une cardiomyopathie hypertrophique obstructive ou d'une hypertrophie due à une surcharge de pression ; ou
pour prendre une décision concernant la thérapie d'un sujet souffrant d'une hypertrophie ventriculaire gauche de type pathologique et/ou pour surveiller ladite thérapie.

**15.** Utilisation d'un kit conçu pour la mise en oeuvre du procédé selon l'une quelconque des revendications 1 à 13, comprenant :

- un anticorps pour déterminer la quantité de troponine T ;
- un anticorps pour déterminer la quantité d'un marqueur de type BNP, en particulier du BNP ou du NT-proBNP ;
- un anticorps pour déterminer la quantité du GDF-15 ;
- des moyens pour comparer les quantités déterminées à l'étape a) à des quantités de référence desdits procédés ; et
- des instructions pour la mise en oeuvre dudit procédé de la présente invention ;

pour diagnostiquer ou discerner, chez un sujet victime d'une hypertrophie ventriculaire gauche, le fait de savoir si le sujet est victime d'une hypertrophie ventriculaire gauche de type physiologique ou souffre d'une hypertrophie ventriculaire gauche de type pathologique ;

pour discerner, chez un sujet souffrant d'une hypertrophie ventriculaire gauche de type pathologique, le fait de savoir si le sujet souffre d'une cardiomyopathie hypertrophique non obstructive, d'une cardiomyopathie hypertrophique obstructive ou d'une hypertrophie due à une surcharge de pression ; ou

pour prendre une décision concernant la thérapie d'un sujet souffrant d'une hypertrophie ventriculaire gauche de type pathologique et/ou pour surveiller ladite thérapie.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 02089657 A
- WO 02083913 A
- EP 0648228 B1
- WO 9906445 A
- WO 0070051 A
- WO 2005113585 A
- US 5744305 A

### Non-patent literature cited in the description

- **LOWBEER et al.** Serum cardiac troponin T, troponin I, plasma BNP and left ventricular mass index in professional football players. *JOURNAL OF SCIENCE AND MEDICINE IN SPORT, SPORTS MEDICINE AUSTRALIA, BELCONNEN,* 24 August 2007, vol. 10 (5), 291-296
- Plasma amino-terminal pro-B-type natriuretic peptide quantification in hypertrophic cardiomyopathy. **ARTEAGA et al.** AMERICAN HEART JOURNAL. MOSBY-YEAR BOOK INC, 01 December 2005, vol. 150, 1228-1232
- **XU JIAN et al.** GDF15/MIC-1 functions as a protective and antihypertrophic factor released from the myocardium in association with SMAO protein activation. *CIRCULATION RESEARCH,* 17 February 2006, vol. 98 (3), 342-350
- **DOWDY ; WEARDEN.** Statistics for Research. John Wiley & Sons, 1983
- **BONOW.** *Circulation,* 1996, vol. 93, 1946-1950
- **SMITH.** *J Endocrinol.,* 2000, vol. 167, 239-46
- **MUELLER.** *Clin Chem Lab Med,* 2004, vol. 42, 942-4
- **WU.** *Clin Chem,* 2004, vol. 50, 867-73
- **SMITH ; WATERMAN.** *Add. APL. Math.,* 1981, vol. 2, 482
- **NEEDLEMAN ; WUNSCH.** *J. Mol. Biol.,* 1970, vol. 48, 443
- **PEARSON ; LIPMAN.** *Proc. Natl. Acad Sci.,* 1988, vol. 85, 2444
- **KARL.** *Scand J Clin Lab Invest,* 1999, vol. 230, 177-181
- **YEO.** *Clinica Chimica Acta,* 2003, vol. 338, 107-115
- **ANDERSON.** *Circulation Research,* 1995, vol. 76 (4), 681-686
- **FERRIERES.** *Clinical Chemistry,* 1998, vol. 44, 487-493
- **ANDERSON ; FERRIERES.** *Clinical Chemistry,* 1998, vol. 44, 487-493
- **MOSES et al.** *PNAS USA,* 1999, vol. 96 (6), 2645-2650
- **BOOTCOV ; HROMAS.** *Biochim Biophys Acta,* 1997, vol. 1354, 40-44
- **LAWTON.** *Gene,* 1997, vol. 203, 17-26
- **YOKOYAMA-KOBAYASHI.** *J Biochem,* 1997, vol. 122, 622-626
- **PARALKAR.** *J Biol Chem,* 1998, vol. 273, 13760-13767
- **BAUSKIN.** *Embo J,* 2000, vol. 19, 2212-2220
- **BOTTNER.** *Gene,* 1999, vol. 237, 105-111
- **BOOTCOV ; TAN ; BAEK.** *Mol Pharmacol,* 2001, vol. 59, 901-908
- **HROMAS ; PARALKAR ; MORRISH.** *Placenta,* 1996, vol. 17, 431-441
- **HANLEY et al.** *Radiology,* 1982, vol. 143, 29-36
- *Am J. Kidney Dis,* 2002, vol. 39 (1
- **NYBERG.** *Cancer Res,* 2005, vol. 65, 3967-3979
- **KHURANA.** *Circulation,* 2005, vol. 111, 2828-2836
- **VAN LAAKE.** *Circulation,* 2006, vol. 114, 2288-2297
- **BOBIK.** *Arterioscler Thromb Vasc Biol,* 2006, vol. 26, 1712-1720
- **BERTOLINO.** *Chest Supplement,* 2005, vol. 128, 585-590
- **MAQLIONE.** *Oncogene,* 1993, vol. 8 (4), 925-31
- Heart Disease. Elsevier Sounders, 2005
- **NOLAN.** *Trends Biotechnol.,* 2002, vol. 20 (1), 9-12